# EUROPÄISCHE PATENTSCHRIFT

(11) **EP 4 404 120 B1**
(45) Veröffentlichungstag und Bekanntmachung des Hinweises auf die Patenterteilung: **30.07.2025**
(21) Anmeldenummer: 23151935.6
(22) Anmeldetag: 17.01.2023
(51) Int. Cl.: G06Q 10/087, B65B 5/10, G16H 20/10

(54) **VERFAHREN ZUR KOMMISSIONIERUNG VON REZEPTPFLICHTIGEN APOTHEKENARTIKELN**
METHOD FOR COMMISSIONING PHARMACY PRODUCTS THAT ARE SUBJECT TO PRESCRIPTION
PROCÉDÉ DE PRÉPARATION DE COMMANDES D'ARTICLES DE PHARMACIE SUR ORDONNANCE

(43) Veröffentlichungstag der Anmeldung: 24.07.2024
(73) Patentinhaber: apo.com Group GmbH, 04416 Markkleeberg (DE)
(72) Erfinder: HEILMANN, Kevin, 04109 Leipzig (DE)
(74) Vertreter: Maikowski & Ninnemann Patentanwälte Partnerschaft mbB

(56) Entgegenhaltungen:
- EP-A1- 2 830 977
- EP-B1- 2 830 977
- DE-T2- 60 308 774
- US-A1- 2010 198 401
- BERGEN MARTIN ET AL: "securPharm e. V. - a protective shield against falsified medicines", BUNDESGESUNDHEITSBLATT - GESUNDHEITSFORSCHUNG - GESUNDHEITSSCHUTZ, SPRINGER BERLIN HEIDELBERG, BERLIN/HEIDELBERG, vol. 60, no. 11, 19 September 2017 (2017-09-19), pages 1255 - 1260, XP036534472, ISSN: 1436-9990, [retrieved on 20170919], DOI: 10.1007/S00103-017-2628-4

## Beschreibung

Die vorliegende Erfindung betrifft ein vollautomatisches Verfahren zur Kommissionierung von rezeptpflichtigen Apothekenartikeln bei dem durch eine Etikettierung der rezeptpflichtigen Apothekenartikel, die kommissioniert werden, eine Identifizierung der rezeptpflichtigen Apothekenartikel und Zuordnung dieser zu einem Kommissionierauftrag zu verschiedenen Zeitpunkten ermöglicht wird. Weiterhin umfasst die Erfindung eine Vorrichtung zur Durchführung des erfindungsgemäßen Verfahrens zur Kommissionierung von rezeptpflichtigen Apothekenartikeln.

Sortieren und Kommissionieren sind wichtige Arbeitsschritte in einem Warenlager, um Artikel effizient in einem Warenlager einzulagern und Artikel aus dem Warenlager entsprechend von Kommissionierlisten zum Beispiel für einen Versand zusammenzustellen. Die einzelnen Schritte sollen dabei möglichst schnell von statten gehen, um einen möglichst effizienten und damit ökonomischen Arbeitsablauf zu gewährleisten.

Eine neue Herausforderung, die gleichzeitig neue Möglichkeiten in der Kommissionierung von Apothekenartikeln eröffnet ist die Einführung des elektronischen Rezepts. Insbesondere im Bereich der Online-Apotheken stellen sich in diesem Zusammenhang neue Aufgaben. Besonders wünschenswert ist die Kommissionierung von Apothekenartikeln möglichst vollautomatisch ablaufen zu lassen, um so den Einsatz von Personal zu verringern und die Arbeitsabläufe möglichst zeiteffizient zu gestalten.

Besonderes Augenmerk muss insbesondere auf die Fehlerfreiheit der Vorrichtungen und Verfahren beim Kommissionieren gelegt werden, da das versehentliche Kommissionieren von falschen Artikeln unbedingt vermieden werden muss. Das Kommissionieren von falschen Artikeln bedeutet dabei, dass Artikel für den Versand zusammengestellt werden, die nicht auf der Kommissionierliste vorhanden sind. Besonders wichtig ist dies im Bereich von rezeptpflichtigen Apothekenartikeln, bei denen aufgrund von falscher Kommissionierung gesundheitliche Risiken für einen Endverbraucher entstehen können.

Automatisierte bzw. halbautomatisierte Kommissionierverfahren sind aus dem Stand der Technik im Bereich der niedergelassenen Apotheken bereits bekannt. Hier werden die gewünschten Medikamente durch einen Apotheker in einem elektronischen Verwaltungssystem angefordert und anschließend vollautomatisch von einem Lagersystem aus einem Lager über eine Fördereinrichtung, wie beispielsweise Röhrensysteme, zum Apotheker geliefert. Eine Kontrolle ob die angeforderten Medikamente den Medikamenten entsprechen, die vom Kommissioniersystem geliefert wurden, erfolgt in dem Fall durch den Apotheker, bevor dieser die Medikamente an einen Kunden ausgibt.

Im Bereich der Online-Apotheken wäre es wünschenswert, eine Kontrolle durch Fachpersonal weitgehend zu reduzieren und nach Möglichkeit eine vollautomatische Kommissionierung zu ermöglichen. Dabei muss jedoch eine fehlerfreie Kommissionierung gewährleistet werden.

Aus dem Stand der Technik sind Verfahren bekannt, bei denen beim Kommissionieren von Artikeln eine Sichtkontrolle durch einen Mitarbeiter erfolgt. Diese Verfahren sind jedoch langsam und haben den Nachteil, dass ein Mitarbeiter mit gleichbleibend hohem Aufmerksamkeitsgrad einer eher eintönigen Arbeit nachgeht. Solche Arbeitsschritte bergen immer die Gefahr von menschlichen Fehlern aufgrund von sinkender Aufmerksamkeit mit der Zeit.

Die WO 2019/135898 offenbart eine Packstation, bei der die Apothekenartikel aus einem Warenlager zu Packtischen transportiert werden, an denen diese dann durch einen Mitarbeiter verpackt werden. Diese Packstationen benötigen zahlreiche ausgebildete Mitarbeiter, was sehr kostenintensiv ist.

Die US10643169 B2 beschreibt ein Kommissionierverfahren bei dem ebenfalls Artikel durch einen Mitarbeiter verpackt werden. Die Verpackten Artikel erhalten eine Kennung, durch die sie später beispielsweise durch eine Kamera identifiziert werden können. Auch hier ist der umfangreiche Einsatz von Mitarbeitern nötig. Eine automatische Identifizierung von rezeptpflichtigen Apothekenartikeln an unterschiedlichen Stellen des Kommissioniervorganges ist hingegen nicht vorgesehen.

Weiterhin sind Verfahren bekannt, bei denen die Aufnahme von Bildern eines Artikels beim Kommissionieren mit mehreren Kameras aus genau festgelegten Positionen vorgesehen ist. Die Artikel befinden sich dabei jeweils auf einer festen Auflage (Fließband o.ä.) oder werden durch einen Roboter den Kameras präsentiert. Die EP3571020B1 offenbart beispielsweise die Anordnung von zwei Kameras an einem Fließband und zumindest einer Kamera an einem Roboterarm. Die WO2019/067174A1 offenbart eine Anordnung von 4 Kameras zur Erkennung von zylinderförmigen Artikeln (z.B. Flaschen) während der Artikel auf einer rotierenden Unterlage (zum Drehen des Artikels) gelagert ist. Alternativ kann der Artikel auch durch einen Roboter einer Kamera präsentiert werden. Die EP2203908B1 offenbart die Anordnung von 3 Kameras, die aus 2 Winkeln Bilder von einem Artikel aufnehmen können. Alle diese Verfahren haben den Nachteil, das entweder nur Bilder aus einem bestimmten Blickwinkel aufgenommen werden und damit nicht der gesamte Artikel erfasst werden kann und/oder das diese sehr langsam sind. Die Geschwindigkeit beim Kommissionieren insbesondere bei der Nutzung von Robotern ist unerwünscht langsam, da das Aufnehmen eines Artikels durch einen Greifarm eines Roboters viel Zeit in Anspruch nimmt.

DE 603 08 774 T2 und US 2010/198401 A1 offenbaren ein System und Verfahren zum automatischen Kommissionieren.

EP 2 830 977 A1 offenbart ein Zentralbandsystem einer Kommissionieranlage mit einem Kamerasystem, das einen Artikel mit mindestens 6 Kameras von allen Seiten erfasst.

Die Aufgabe der vorliegenden Erfindung ist es daher die Nachteile der aus dem Stand der Technik bekannten Verfahren zu beheben und ein Kommissionierverfahren zur Verfügung zu stellen, welches vollautomatisch abläuft und dabei eine fehlerfreie Kommissionierung von rezeptpflichtigen Medikamenten ermöglicht. Das Verfahren soll dabei eine schnelle Kommissionierung ermöglichen.

Hierfür stellt die vorliegende Erfindung ein Verfahren gemäß Anspruch 1 und eine Vorrichtung gemäß Anspruch 12 zur Verfügung.

### Detaillierte Beschreibung

### Rezeptpflichtige Apothekenartikel

Erfindungsgemäß werden rezeptpflichtige Apothekenartikel aus einem Warenlager in Packkartons kommissioniert. Für jeden zu kommissionierenden rezeptpflichtigen Apothekenartikel ist ein SecurPharm Code bekannt, der auf dem rezeptpflichtigen Apothekenartikel oder dessen Umverpackung aufgebracht ist. Erfindungsgemäß wird weiterhin für jeden zu kommissionierenden rezeptpflichtigen Apothekenartikel ein Etikettencode erstellt, mit dem der Apothekenartikel gekennzeichnet wird.

Jeder Apothekenartikel weist mindestens einen Identifikationscode auf, der vom Hersteller, bei der Erzeugung des Artikels, auf der Umverpackung aufgebracht wird. Grundlegend weist jede Umverpackung eines Apothekenartikels in Klarschrift die individuellen Erkennungsmerkmale des Apothekenartikels in Form der Seriennummer, der Pharmazentralnummer, der Chargennummer und des Verfallsdatums auf. Darüber hinaus werden diese Daten bei rezeptpflichtigen Apothekenartikeln in einem Data Matrix Code codiert auf die Umverpackung gedruckt. Dieser Data Matrix Code verschlüsselt den sogenannten SecurPharm Code, der der Verifikation des rezeptpflichtigen Apothekenartikels bei der Abgabe an einen Verbraucher dient. Um die europäische Fälschungsrichtlinie 2011/62/EU umzusetzen, betreibt die European Medicines Verification Organisation (EMVO) ein europäisches Netzwerk (EU-Hub), welches der Echtheitsprüfung von rezeptpflichtigen Apothekenartikeln dient. Dabei werden die individuellen Erkennungsmerkmale eines rezeptpflichtigen Apothekenartikels bei der Herstellung in dem Netzwerk hinterlegt. Bei der Abgabe eines rezeptpflichtigen Apothekenartikels an einen Verbraucher wird der SecurPharm Code auf der Umverpackung ausgelesen und die darin enthaltenen individuellen Erkennungsmerkmalen des rezeptpflichtigen Apothekenartikels werden über den EU-Hub mit den bei der Herstellung hinterlegten individuellen Erkennungsmerkmalen abgeglichen. Anschließend werden die individuellen Erkennungsmerkmale des an den Verbraucher abgegebenen Apothekenartikels aus dem EU-Hub abgemeldet. Wird bei dem Abgleich jedoch festgestellt, dass die individuellen Erkennungsmerkmalen des rezeptpflichtigen Apothekenartikels bereits abgemeldet sind, wird der Artikel nicht an den Verbraucher abgegeben, sondern zur Sicherheit des Verbrauchers einbehalten und der Vorfall überprüft.

Weiterhin weisen alle Apothekenartikel und sonstige apothekenüblichen Produkte eine Pharmazentralnummer auf. Die Pharmazentralnummer (PZN) ist ein in Deutschland bundeseinheitlicher Identifikationsschlüssel für Arzneimittel und sonstige apothekenüblichen Produkte. Sie kennzeichnet Arzneimittel eindeutig nach Bezeichnung, Darreichungsform, Wirkstoffstärke und Packungsgröße. Die PZN-Nummer wird im Klartext und als Strichcode auf der Umverpackung der Arzneimittel aufgebracht.

In einer Ausführungsform der vorliegenden Erfindung weist der rezeptpflichtige Apothekenartikel mehr als einen Identifikationscode des Herstellers auf. Der weitere Identifikationscode des Herstellers kann dann die PZN-Nummer sein.

Die Position des SecurPharm Codes auf dem rezeptpflichtigen Apothekenartikel ist erfindungsgemäß bekannt. Diese Information kann beispielsweise beim Einlagern eines rezeptpflichtigen Apothekenartikels in ein Lager gewonnen werden und dann in einer Datenbank hinterlegt werden.

Erfindungsgemäß wird für jeden zu kommissionierenden Apothekenartikel ein Etikettencode erzeugt. Der Etikettencode umfasst die Bestellnummer des rezeptpflichtigen Apothekenartikels und weiterhin Informationen ausgewählt aus der Gruppe aufweisend mindestens einen Identifikationscode des Herstellers, PZN-Nummer, Chargennummer, Seriennummer, Verfallsdatum. Der Etikettencode umfasst erfindungsgemäß eine eindeutige Zeichenfolge zur Identifikation des Produktes. Der Etikettencode liegt erfindungsgemäß in Form eines Data Matrix Codes vor.

In einer Ausführungsform der Erfindung setzt sich der Etikettcode aus einen Zeichenfolge mit einem Führungsbuchstaben zu Beginn und 29 numerischen Zeichen im Anschluss zusammen.

Erfindungsgemäß wird mindestens ein analoges oder digitales Rezept zur Verfügung gestellt. Das elektronische Rezept kann beispielsweise über eine geeignete digitale Plattform oder eine App übermittelt werden. Weiterhin wird ein digitaler Kommissionierauftrag für das elektronische Rezept erstellt. Dem digitalen Kommissionierauftrag werden alle für den Kommissionierauftrag des elektronischen Rezeptes relevante digitalen Daten währen des Ablaufs des gesamten Kommissionierverfahrens zugeordnet und diese gespeichert. Liegt ein analoges Rezept vor, wird dieses zunächst digitalisiert. Anschließend wir analog zum digitalen Rezept verfahren.

Erfindungsgemäß wird demnach das mindestens eine analoge oder digitale Rezept eingelesen.

Dabei werden folgende Daten erkannt
- die auf dem mindestens einen Rezept aufgelisteten rezeptpflichtigen Apothekenartikel;
- personenbezogenen Daten des Rezeptempfängers auf dem elektronischen Rezept, wie Name, Adresse, Krankenkasse;
- Dosierungsanweisungen;
- weiterer Hinweise des Arztes, die auf dem elektronischen Rezept vermerkt sind; und
- optional auf dem mindestens einem Rezept aufgelisteten nichtrezeptpflichtige Apothekenartikel.

Diese Daten werden dem digitalen Kommissionierauftrag zugeordnet. Darüber hinaus können dem digitalen Kommissionierauftrag noch weitere Daten zugeordnet werden, wie beispielsweise:
- Kundennummer;
- Auftragsdatum;
- Name, Adresse, Telefonnummer der abgebenden Apotheke;
- Weitere Bestellungen des Kunden von nichtrezeptpflichtigen Medikamenten und/oder Drogeriewaren.

Optional wird mindestens ein weiteres analoges oder digitales Rezept bereitgestellt. In einer Ausführungsform wird nicht nur ein weiteres analoges oder digitales Rezept, sondern werden n weitere analoge oder digitale Rezepte bereitgestellt, n ∈ N. Dieses mindestens eine weitere analoge oder digitale Rezept wird wie bereits beschrieben bearbeitet und dessen Daten dem digitalen Kommissionierauftrag zugeordnet und mit diesem verknüpft.

In einem weiteren Verfahrensschritt wird ein rezeptpflichtiger Apothekenartikel des digitalen Kommissionierauftrages aus einem Warenlager durch ein Handhabungsgerät und/oder einen Roboter und/oder eine Fördereinrichtung bereitgestellt. Das heißt, es wird ein rezeptpflichtiger Apothekenartikel bereitgestellt, der auf dem mindestens einen analogen oder digitalen Rezept aufgeführt ist und daher im digitalen Kommissionierungsauftrag aufgeführt wird. Das Warenlager, aus dem der rezeptpflichtige Apothekenartikel bereitgestellt wird, kann prinzipiell jede Art von Lagersystem aufweisen, beispielsweise Lagersysteme nach dem ABC-Prinzip oder auch Lagersysteme nach dem chaotischen Prinzip. In einer besonders bevorzugten Ausführungsform ist das Warenlager ein sogenannter KNAPP-Store. KNAPP-Store ist ein bekanntes Lagersystem der Firma KNAPP AG, welches nach dem chaotischen Prinzip einlagert und sich selbst optimiert. Wie bereits beschrieben ist mindestens ein Identifikationscode des Herstellers zu dem ausgelagerten rezeptpflichtigen Apothekenartikel bekannt, dieser wird dem digitalen Kommissionierauftrag zusammen mit der Bestellnummer des rezeptpflichtigen Apothekenartikels zugeordnet. Der mindestens eine Identifikationscode des Herstellers und die Bestellnummer können zum Beispiel in einer Datenbank hinterlegt sein.

In einer Ausführungsform der Erfindung weist das erfindungsgemäße Verfahren zusätzliche Schritte auf, in denen rezeptpflichtige Apothekenartikel in ein geeignetes Warenlager, beispielsweise in einen KNAPP-Store einsortiert werden. Beim Einsortieren der rezeptpflichtigen Apothekenartikel werden diese gescannt und mindesten ein Identifikationscode des Herstellers wird erkannt. Der Ablageort im Warenlager für jeden der sortierten rezeptpflichtigen Apothekenartikel ist ebenfalls bekannt und kann beispielsweise in einer Datenbank verknüpft mit dem mindesten einen Identifikationscode des Herstellers hinterlegt werden.

Der Etikettcode kann beim Einlagern bereits erzeugt werden oder aber auch erst beim Bereitstellen des rezeptpflichtigen Apothekenartikels.

Erfindungsgemäß wird ein Etikett gedruckt. Das Etikett weist
- personenbezogene Daten des Rezeptempfängers;
- Daten der ausgebenden Apotheke, wie Name, Adresse, Telefonnummer;
- Angaben zur Dosierung des bereitgestellten rezeptpflichtigen Apothekenartikels;
- einen DataMatrix-Code, aufweisend den Etikettcode des bereitgestellten rezeptpflichtigen Apothekenartikels; und
- optional weiterer Hinweise des Arztes, die auf einem Rezept bezüglich des rezeptpflichtigen Apothekenartikels vermerkt sind;
auf. Die Daten, die auf das Etikett gedruckt werden, sind durch den digitalen Kommissionierauftrag bekannt und stehen zur Verfügung.

Das Etikett wird erfindungsgemäß derart auf einer Seite des rezeptpflichtigen Apothekenartikels appliziert, dass das Etikett den SecurPharm Code des rezeptpflichtigen Apothekenartikels nicht verdeckt. Dies ist möglich, da dessen Position auf dem rezeptpflichtigen Apothekenartikel bekannt ist.

In einer Ausführungsform der Erfindung weist das erfindungsgemäße Verfahren wie bereits beschrieben zusätzliche Schritte auf, in denen rezeptpflichtige Apothekenartikel in ein geeignetes Warenlager, beispielsweise in einen KNAPP-Store einsortiert werden. Beim Einsortieren der rezeptpflichtigen Apothekenartikel werden diese gescannt, wobei die Position mindestens eines Identifikationscodes, insbesondere des Data Matrix Codes der den SecurPharm Code beinhaltet, auf dem rezeptpflichtigen Apothekenartikel oder dessen Umverpackung erkannt wird. Diese Information kann dann beispielsweise in einer Datenbank zugehörig zur Identifikationsnummer des Herstellers, der Ablageposition des rezeptpflichtigen Apothekenartikels im Warenlager und dem Gewicht des rezeptpflichtigen Apothekenartikels in einer Datenbank gespeichert werden. Diese Datenbank kann bevorzugt zum verwendeten Warenlager gehören.

Zur Überprüfung wird der DataMatrix-Code des Etiketts anschließend eingescannt und der darin enthaltene Etikettcode ausgelesen. Der Etikettcode wird mit den im digitalen Kommissionierauftrag hinterlegten Etikettcodes verglichen. Wird eine Übereinstimmung mit einem im digitalen Kommissionierauftrag hinterlegten Etikettcode festgestellt, wird der rezeptpflichtige Apothekenartikel in einer leeren Pufferschale abgelegt. Wird hingegen keine Übereinstimmung zu einem im digitalen Kommissionierauftrag hinterlegten Etikettcode festgestellt, so wird der etikettierte rezeptpflichtige Apothekenartikel in einer Fehlerschale abgelegt.

Auf diese Weise kann sichergestellt werden, dass der bereitgestellte rezeptpflichtige Apothekenartikel tatsächlich zum digitalen Kommissionierauftrag gehört. Fehler bezüglich der Identität des rezeptpflichtigen Apothekenartikels beim Etikettieren werden dadurch vorteilhafterweise ausgeschlossen.

Die Schritte
- Bereitstellen eines rezeptpflichtigen Apothekenartikels, des digitalen Kommissionierauftrages aus einem Warenlager durch eine Handhabungsgerät und/oder einen Roboter und/oder eine Fördereinrichtung;
- Zuordnen mindestens eines Identifikationscodes des Herstellers des bereitgestellten rezeptpflichtigen Apothekenartikels zu dem digitalen Kommissionierauftrag;
- Drucken eines Etiketts, aufweisend
   o personenbezogene Daten des Rezeptempfängers;
   ∘ Daten der ausgebenden Apotheke, wie Name, Adresse, Telefonnummer;
   ∘ Angaben zur Dosierung des bereitgestellten rezeptpflichtigen Apothekenartikels;
   o einen DataMatrix-Code, aufweisend den Etikettcode des bereitgestellten rezeptpflichtigen Apothekenartikels; und
   ∘ optional weiterer Hinweise des Arztes, die auf dem elektronischen Rezept vermerkt sind;
- Applizieren des Etiketts auf einer Seite des rezeptpflichtigen Apothekenartikels derart, dass das Etikett den SecurPharm Code des rezeptpflichtigen Apothekenartikels nicht verdeckt;
- Einscannen des DataMatrix-Codes des Etiketts und auslesen des darin enthaltenen Etikettcodes und Vergleich des Etikettcodes, mit den im digitalen Kommissionierauftrag enthaltenen Etikettcodes;
- Ablegen des etikettierten rezeptpflichtigen Apothekenartikels in einer leeren Pufferschale, wenn eine Übereinstimmung mit einem im digitalen Kommissionierauftrag hinterlegten Etikettcode festgestellt wird und ablegen des etikettierten rezeptpflichtigen Apothekenartikels in einer Fehlerschale, wenn keine Übereinstimmung mit einem im digitalen Kommissionierauftrag hinterlegten Etikettcode festgestellt wird;
werden erfindungsgemäß für alle rezeptpflichtigen Apothekenartikel, die dem digitalen Kommissionierauftrag zugeordnet sind, wiederholt. Hierdurch können alle durch das mindestens eine Rezept und jedes weitere bereitgestellte Rezept angeforderten rezeptpflichtigen Apothekenartikel eines digitalen Kommissionierauftrages aus dem Warenlager entnommen und in Pufferschalen oder gegebenenfalls in Fehlerschalen abgelegt werden.

Beim Erstellen des Etiketts oder beim Etikettieren können folgende Fehler auftreten:
- Es wird ein falsches Etikett gedruckt, das heißt die Daten des Etiketts stimmen nicht mit den Daten des digitalen Kommissionierauftrages überein. Dieser Fehler würde beim Einscannen des Etiketts eines rezeptpflichtigen Apothekenartikels vor der Abgabe in eine Pufferschale erkannt werden. In diesem Fall wird, wie beschrieben der rezeptpflichtige Apothekenartikel in eine Fehlerschale abgegeben.
- Es kann kein Etikett gedruckt werden, beispielsweise weil keine Etiketten mehr da sind und/oder der Drucker funktionsuntüchtig ist. In diesem Fall wird eine Fehlermeldung erzeugt. Die Fehlermeldung kann auf elektronischem Weg und/oder durch ein akustisches und/oder visuelles Signal ausgegeben werden. Zur Fehlerbehebung ist an dieser Stelle der Eingriff eines Mitarbeiters notwendig.
- Das Etikett passt nicht auf den rezeptpflichtigen Apothekenartikel, weil dessen Maße kleiner sind als die Maße des Etiketts. In diesem Fall wird eine Fehlermeldung erzeugt. Der rezeptpflichtige Apothekenartikel wird in eine leere Pufferschale abgelegt und diese wird in eine Kontrollstation geleitet, in welcher der Apothekenartikel händisch von einem Mitarbeiter mit einem geeigneten Etikett versehen wird. Der dann mit einem Etikett versehene Apothekenartikel wird anschließend den im Folgenden beschriebenen Verfahrensschritten wieder zugeführt.
- Das Etikett kann nicht eingescannt werden, das heißt aus welchen Gründen auch immer kann der DataMatrix-Code des Etiketts nicht gelesen werden. In diesem Fall wird der rezeptpflichtige Apothekenartikel in eine Fehlerschale abgegeben.

Bei allen genannten Fehlern geht der Drucker zusätzlich in eine Funktionsstörung über. Hierdurch wird der weitere Kommissioniervorgang für nachfolgende Aufträge unterbrochen. Eine Funktionskontrolle und Fehlerbehebung werden dann durch einen Mitarbeiter vorgenommen.

Wird ein rezeptpflichtiger Apothekenartikel in der Fehlerschale abgelegt und kann damit nicht kommissioniert werden, so wird in einer Ausführungsform der Kommissioniervorgang abgebrochen und eine Fehlermeldung erzeugt. Die Fehlermeldung kann auf elektronischem Weg und/oder durch ein akustisches und/oder visuelles Signal ausgegeben werden. Zur Fehlerbehebung ist an dieser Stelle der Eingriff eines Mitarbeiters notwendig. In einer weiteren Ausführungsform wird ein weitere rezeptpflichtiger Apothekenartikel der gleichen Sorte bereitgestellt und durch einen anderen Drucker entsprechend dem erfindungsgemäßen Verfahren etikettiert und den weiteren Verfahrensschritten zugeführt.

Konnten alle rezeptpflichtigen Apothekenartikel des digitalen Kommissionierauftrages verfahrensgemäß in Pufferschalen abgelegt werden, werden nacheinander die rezeptpflichtigen Apothekenartikel aus den Pufferschalen auf eine Fördertechnik ausgegeben. Dies kann zum Beispiel durch das Öffnen oder Auskippen der Pufferschalen auf eine Fördertechnik erfolgen. Geeignete Fördertechnik ist zum Beispiel ein Förderband. Der Inhalt der einzelnen Pufferschalen wird nacheinander ausgegeben, so dass jeder rezeptpflichtige Apothekenartikel auf der Fördertechnik mindestens einen vorgegebenen Abstand zu einem weiteren rezeptpflichtigen oder nichtrezeptpflichtigen Apothekenartikel aufweist. Dies kann erreicht werden, indem der Inhalt verschiedener Pufferschalen zeitlich beabstandet auf die Fördertechnik abgegeben wird oder in dem verschiedene Pufferschalen räumlich beabstandet voneinander angeordnet sind, so dass bei der zeitgleichen Abgabe des Inhalts verschiedener Pufferschalen auf eine Fördertechnik, insbesondere ein Förderband, der Inhalt verschiedener Pufferschalen mit einem vorgegebenen Mindestabstand auf der Fördertechnik zu liegen kommen. Erfindungsgemäß beträgt der Abstand zwischen einem rezeptpflichtigen Apothekenartikel einem darauffolgenden Gegenstand auf einem Förderband mindestens 30 cm, bevorzugt beträgt der Abstand zwischen 30 cm und 50 cm.

Die rezeptpflichtigen Apothekenartikel werden erfindungsgemäß durch die Fördertechnik zu einer Packstation transportiert.

An der Packstation wird ein etikettierter Packkarton bereitgestellt. Das Etikett des Packkartons weist eine Karton-Identifikationsnummer auf, die eingescannt wird und dem digitalen Kommissionierauftrag zugeordnet wird.

Erfindungsgemäß wird weiterhin ein auf der Fördertechnik transportierter rezeptpflichtiger Apothekenartikel in einen Trichter abgegeben. Die Öffnung am Hals des Trichters ist dabei verschlossen, so dass der Trichter zunächst als Sammelbehälter dienen kann. Unterhalb des Halses des Trichters ist der bereitgestellte etikettierte Packkarton positioniert. Fällt ein rezeptpflichtiger Apothekenartikel von der Fördereinrichtung in den Trichter wird dieser nach Verlassen der Fördereinrichtung, während des Falles in der Luft und damit vor dem Auftreffen im inneren des Trichters, von 6 Seiten gescannt. Beim Scannen wird der DataMatrix-Code des Etiketts des rezeptpflichtigen Apothekenartikels erkannt und der SecurPharm Code auf dem rezeptpflichtigen Apothekenartikel oder dessen Umverpackung. Das Scannen erfolgt bevorzugt mit Digitalkameras. Hierfür sind am Ende der Fördertechnik mindestens 6 Digitalkameras derart positioniert, dass von dem in den Trichter fallenden rezeptpflichtigen Apothekenartikel von 6 unterschiedlichen Seiten Bilder aufgenommen werden können. Bevorzugt sind die Digitalkameras derart positioniert, dass die Digitalkameras an zwei unterschiedlichen Positionen, gegenüberliegende Seiten des rezeptpflichtigen Apothekenartikels abbilden können.

Dabei wird in einer Ausführungsform der Erfindung an einem Rahmen eine erste Digitalkamera oberhalb der Fördertechnik, eine zweite Digitalkamera rechts und eine dritte Digitalkamera links der Fördertechnik positioniert. Eine vierte Digitalkamera ist so positioniert, dass sie Bilder von dem rezeptpflichtigen Apothekenartikel aufnehmen kann, nachdem dieser die vierte Digitalkamera passiert hat. Eine fünfte Digitalkamera ist derart positioniert, dass sie Bilder von dem rezeptpflichtigen Apothekenartikel aufnehmen kann, während dieser sich auf der Fördertechnik auf die fünfte Digitalkamera zubewegt. Eine sechste Kamera ist derart positioniert, dass sie von unten Bilder von dem rezeptpflichtigen Apothekenartikel aufnehmen kann, während dieser von der Fördertechnik in den Trichter fällt. Die 6 Digitalkameras sind damit an 6 verschiedenen Positionen angeordnet, so dass von 6 unterschiedlichen Seiten Bilder des rezeptpflichtigen Apothekenartikels aufgenommen werden können. Jede Kamera kann maximal Bilder mit einer Frequenz von 55 Hz von dem rezeptpflichtigen Apothekenartikel aufnehmen.

In einer bevorzugten Ausführungsform werden anstatt Digitalkameras Barcodeleser verwendet, die geeignet sind, Data Matrix Codes zu erkennen. Barcodeleser weisen vorteilhafterweise ebenfalls eine ausreichend hohe Bildaufnahmefrequenz auf. Die Anordnung der Barcodescanner entspricht dabei der Anordnung, die für die Digitalkameras bereits beschrieben wurde. Ebenso gilt alles im Folgenden für den Einsatz von Digitalkameras beschriebene gleichermaßen für den Einsatz von Barcodelesern.

Die beschriebene Anordnung der Digitalkameras oder der Barcodeleser für den 6-Seiten Scan an einem Rahmen hat den Vorteil, dass diese Vorrichtung einfach in bereits bestehende Kommissioniervorrichtungen integriert werden kann. Eine Nachrüstung ist vorteilhafterweise ohne wesentliche technische Umbauten möglich.

In einer Ausführungsform der vorliegenden Erfindung ist die Fördertechnik ein Förderband mit einem Seitenschutz, damit keines der transportierten Artikel seitlich vom Förderband fallen kann. In diesem Fall ist es besonders bevorzugt, wenn an dem Ende des Förderbandes, welches sich am Trichter befindet, der Seitenschutz durchsichtig ausgebildet ist. Dies ist besonders vorteilhaft innerhalb des Blickwinkels der Digitalkameras die rechts und links des Förderbandes der Fall. Auf diese Weise wird der Blickwinkel der Digitalkameras nicht durch den Seitenschutz beeinträchtigt.

In einer bevorzugten Ausführungsform wird an den 6 beschriebenen Positionen jeweils nicht nur eine Digitalkamera positioniert, sondern jeweils zwei. Die beiden Digitalkameras einer Position werden beabstandet voneinander angebracht, so dass deren Blickwinkel auf den rezeptpflichtigen Apothekenartikel jeweils ein wenig unterschiedlich ist. Damit werden vorteilhafterweise Bilder aus doppelt so vielen Perspektiven aufgenommen im Vergleich zur Verwendung von einer Digitalkamera pro Position, womit der Informationsgehalt der aufgenommenen Bilder gesteigert werden kann.

Anschließend wird der im gescannten Data Matrix Code des Etiketts enthaltenen Etikettcode und der gescannten SecurPharm Code, der auf dem Artikel an sich bzw. auf dessen Umverpackung aufgebracht ist und die zuvor gescannte Karton-Identifikationsnummer mit den digitalen Daten, die dem digitalen Kommissionierauftrag zugeordnet sind, verglichen.

Anschließend wird der auf der Fördertechnik befindliche nächste rezeptpflichtige Apothekenartikel in den Trichter abgegeben. Dabei wird dieser, wie bereits beschrieben, von 6 Seiten gescannt. Anschließend werden wiederum der im gescannten Data Matrix Code des Etiketts enthaltenen Etikettcode und der gescannten SecurPharm Code, der auf dem Artikel an sich bzw. auf dessen Umverpackung aufgebracht ist und die zuvor gescannte Karton-Identifikationsnummer mit den digitalen Daten, die dem digitalen Kommissionierauftrag zugeordnet sind, verglichen. Die Schritte
- der Abgabe der rezeptpflichtigen Apothekenartikel in den Trichter,
- des Einscannens und des Vergleichens des im gescannten Data Matrix Code des Etiketts enthaltenen Etikettcodes und des gescannten SecurPharm Codes, der auf dem Artikel an sich bzw. auf dessen Umverpackung aufgebracht ist und der zuvor gescannten Karton-Identifikationsnummer mit den digitalen Daten, die dem digitalen Kommissionierauftrag zugeordnet sind,
werden für alle rezeptpflichtigen Artikel durchgeführt, die von dem digitalen Kommissionierauftrag umfasst sind. Alle rezeptpflichtigen Apothekenartikel, die von dem digitalen Kommissionierauftrag umfasst sind, werden erfindungsgemäß in den gleichen Trichter abgegeben.

Das Einscannen der rezeptpflichtigen Apothekenartikel bei der Abgabe dieser in den Trichter kann erfindungsgemäß nacheinander für jeden Artikel einzeln erfolgen, da jeder rezeptpflichtige Apothekenartikel beabstandet zu einem weiteren rezeptpflichtigen Apothekenartikel oder einem anderen Gegenstand auf der Fördertechnik liegt. Hierdurch wird ausgeschlossen, dass von zwei oder noch mehr rezeptpflichtige Apothekenartikel zur gleichen Zeit durch die Digitalkameras Bilder aufgenommen werden, bzw. zwei oder mehr rezeptpflichtige Apothekenartikel auf einmal in den Trichter fallen.

Sind alle mit dem digitalen Kommissionierauftrag verknüpften rezeptpflichtigen Apothekenartikel in dem Trichter, wird der Trichter geöffnet und sämtliche im Trichter gesammelten Artikel fallen in den Packkarton, der unter dem Trichter bereitgestellt ist.

Wenn im Verfahrensschritt p) für alle zu kommissionierenden rezeptpflichtigen Apothekenartikel festgestellt wurde, dass der Etikettcode und der SecurPharm Codes mit einem Etikettcode und einem SecurPharm Code übereinstimmen, die im digitalen Kommissionierauftrag hinterlegt sind und die gescannte Karton-Identifikationsnummer mit der Karton-Identifikationsnummer übereinstimmt, die im digitalen Kommissionierauftrages hinterlegt ist, wird der Freigabe des Packkartons zum Versand oder der weiteren Verarbeitung freigegeben.

Wenn hingegen bei der Abgabe der Artikel in den Trichter eine Abweichung zwischen den gescannten Daten und den Daten des digitalen Kommissionierauftrages erkannt wurde, das heißt, wenn eines der folgenden Ereignisse eintritt:
- zumindest ein gescannter Etikettcode eines rezeptpflichtigen Apothekenartikels ist nicht im digitalen Kommissionierauftrag hinterlegten und/oder
- zumindest eine gescannte SecurPharm Nummer ist nicht im digitalen Kommissionierauftrag hinterlegten; und/oder
- die gescannte Karton-Identifikationsnummer ist nicht im digitalen Kommissionierauftrag hinterlegten; und/oder
- es kann vom Etikett eines rezeptpflichtigen Apothekenartikels kein Etikettcode, z.B. weil das Etikett nicht richtig gescannt werden konnte; und/oder
- es kann von einem rezeptpflichtigen Apothekenartikel kein SecurPharm Code gescannt werden, z.B. weil dieser nicht richtig gescannt wurde;
wird der Packkarton über eine Fördereinrichtung einer Kontrolle zur Überprüfung übergeben.

Erfindungsgemäß wird der SecurPharm Code jedes der in einen Packkarton kommissionierten apothekenpflichtigen Apothekenartikel bei Versand des Kartons automatisch über das EU-Hub Netzwerk abgemeldet, wobei ein Zeitstempel für die Abmeldung erzeugt wird. Damit wird die europäische Richtlinie 2011/62/EU umgesetzt. Die Daten des Etiketts und der Zeitstempel für die Abmeldung über das EU-Hub Netzwerk werden in einer Ausführungsform untereinander und mit dem digitalen Kommissionierauftrag verknüpft und gespeichert. Damit ist eine lückenlose Verfolgung des Abgabeweges eines rezeptpflichtigen Apothekenartikels möglich. Wobei vorteilhaftweise eine vollautomatische Kommissionierung durch das vorliegende Verfahren ermöglicht wird. Durch die unterschiedlichen Kontrollinstanzen wird dabei die Fehlerfreiheit der vollautomatischen Kommissionierung sichergestellt.

### Nichtrezeptpflichtige Apothekenartikel und/oder Drogerieartikel

In einer Ausführungsform der vorliegenden Erfindung werden weiterhin nichtrezeptpflichtige Apothekenartikeln und/oder Drogerieartikeln aus einem Warenlager kommissioniert. Nichtrezeptpflichtige Apothekenartikel können dabei beispielsweise über die im erfindungsgemäßen Verfahren eingelesenen analogen oder digitalen Rezepte bestellt werden oder alternativ zum Beispiel durch einen Einkauf in einem Online-Shop. Ein Online-Shop ermöglicht auch das Bestellen von Drogerieartikeln, die beim Bestellvorgang dem digitalen Kommissionierauftrag zugeordnet werden. Die Bestelldaten, insbesondere die Art und Anzahl der bestellten nichtrezeptpflichtigen Apothekenartikel und/oder Drogerieartikel, Kundendaten wie Name und Adresse, Bestellnummern, Kundennummer werden mit dem digitalen Kommissionierauftrag verknüpft.

Erfindungsgemäß sind für jeden nichtrezeptpflichtigen Apothekenartikel und/oder Drogerieartikel ein oder mehrere Identifikationscodes des Herstellers bekannt, durch die die zu kommissionierenden nichtrezeptpflichtigen Apothekenartikel und/oder Drogerieartikel eindeutig identifiziert werden können, und wobei mindestens ein Identifikationscode des Herstellers auf jedem zu kommissionierenden nichtrezeptpflichtigen Apothekenartikel und/oder Drogerieartikel oder auf dessen Umverpackung aufgebracht ist. Der Identifikationscode des Herstellers kann als 1D Code oder auch al 2D Code aufgebracht sein. Ein geeigneter 1D Code ist beispielsweise ein Strichcode in Form eines Code 128, Code 39, Interleaved 2 of 5, UPC oder EAN während ein 2D Code ein Data Matrix Code sein kann. Der Identifikationscode des Herstellers umfasst dabei bevorzugt die PZN-Nummer des Artikels, wodurch dieser eindeutig Identifiziert werden kann. Erfindungsgemäß wird für jeden zu kommissionierenden nichtrezeptpflichtigen Apothekenartikel und/oder Drogerieartikel mindestens ein Identifikationscode des Herstellers mit dem Kommissionierauftrag verknüpft.

In dieser Ausführungsform weist das erfindungsgemäße Verfahren die folgenden Schritte auf
aa) Bereitstellen eines digitalen oder analogen Rezeptes und Erstellen eines digitalen Kommissionierauftrages für das digitale oder analoge Rezept;
ab) Einlesen des digitalen oder analogen Rezeptes und erkennen der durch das Rezept bestellten rezeptpflichtigen Apothekenartikel, der personenbezogenen Daten auf dem elektronischen Rezept, Dosierungsanweisungen, weiterer Hinweise des Arztes, die auf dem elektronischen Rezept vermerkt sind und erkennen der durch das Rezept bestellten nichtrezeptpflichtigen Apothekenartikel und/oder Drogerieartikel;
ac) Zuordnen der in Schritt ab) erkannten digitalen Daten zu dem digitalen Kommissionierauftrag und optional zuordnen weiterer Bestellungen von nichtrezeptpflichtigen Apothekenartikel und/oder Drogerieartikel;
ad) Optional bereitstellen mindestens eines weiteren digitalen oder analogen Rezeptes und einlesen des mindestens einen weiteren digitalen oder analogen Rezeptes und erkennen der durch das mindestens eine weitere digitale oder analoge Rezept bestellten rezeptpflichtigen Apothekenartikel, der personenbezogenen Daten auf dem mindestens einen weiteren digitalen oder analogen Rezept, Dosierungsanweisungen, weiterer Hinweise des Arztes, die auf dem mindestens einen weiteren digitalen oder analogen Rezept vermerkt sind und erkennen der durch das mindestens eine weitere digitalen oder analogen Rezept bestellten nichtrezeptpflichtigen Apothekenartikel und/oder Drogerieartikel; und Zuordnen der erkannten digitalen Daten zu dem in Schritt aa) erzeugten digitalen Kommissionierauftrag;
ae) Bereitstellen eines rezeptpflichtigen Apothekenartikels, des digitalen Kommissionierauftrages aus einem Warenlager durch einen Roboter und/oder eine Fördereinrichtung;
af) Zuordnen des Etikettcodes des bereitgestellten rezeptpflichtigen Apothekenartikels zu dem digitalen Kommissionierauftrag;
ag) Drucken eines Etiketts, aufweisend personenbezogene Daten des Rezeptempfängers, Angaben zur Dosierung des bereitgestellten rezeptpflichtigen Apothekenartikels, eines DataMatrix-Codes aufweisend den Etikettcode des bereitgestellten rezeptpflichtigen Apothekenartikels und optional weiterer Hinweise des Arztes, die auf einem elektronischen Rezept bezüglich des rezeptpflichtigen Apothekenartikels vermerkt sind;
ah) Applizieren des Etiketts auf einer Seite des rezeptpflichtigen Apothekenartikels derart, dass das Etikett den SecurPharm Code des rezeptpflichtigen Apothekenartikels nicht verdeckt;
ai) Einscannen des Data Matrix Codes des Etiketts und auslesen des darin enthaltenen Etikettcodes und Vergleich des Etikettcodes mit den dem digitalen Kommissionierauftrag zugeordneten Etikettcodes;
aj) Ablegen des etikettierten rezeptpflichtigen Apothekenartikels in einer leeren Pufferschale, wenn im Verfahrensschritt ai) festgestellt wurde, dass der Etikettcode mit einem Etikettcode, der im digitalen Kommissionierauftrag hinterlegt ist, übereinstimmt oder Ablegen des etikettierten rezeptpflichtigen Apothekenartikels in einer Fehlerschale, wenn im Verfahrensschritt ai) festgestellt wurde, dass der Etikettcode nicht mit einem Etikettcode, der im digitalen Kommissionierauftrag hinterlegt ist, übereinstimmt;
ak) Wiederholen der Schritte ae) bis aj) für alle rezeptpflichtigen Apothekenartikel, die dem digitalen Kommissionierauftrag zugeordnet sind;
al) Bereitstellen aller im digitalen Kommissionierauftrag enthaltenen nichtrezeptpflichtigen Apothekenartikel und/oder Drogerieartikel in einer Pufferschale;
am) Nacheinander Ausgabe der rezeptpflichtigen Apothekenartikel aus den Pufferschalen auf eine Fördertechnik, so dass jeder rezeptpflichtige Apothekenartikel auf der Fördertechnik mindestens einen vorgegebenen Abstand zu einem weiteren rezeptpflichtigen Apothekenartikel oder einem anderen Artikel aufweist und Transport der rezeptpflichtigen Apothekenartikel durch die Fördertechnik zu einer Packstation;
an) Abgabe der nichtrezeptpflichtigen Apothekenartikel und/oder Drogerieartikel als Haufen aus der Pufferschale auf die Fördertechnik aus Verfahrensschritt am);
ao) Bereitstellen eines etikettierten Packkartons, wobei das Etikett des Packkartons eine Karton-Identifikationsnummer aufweist und Zuordnen der Karton-Identifikationsnummer zu dem digitalen Kommissionierauftrag;
ap) Scannen der Karton-Identifikationsnummer vom Etikett des Packkartons;
aq) Abgabe eines auf der Fördertechnik transportierten rezeptpflichtigen Apothekenartikels in einen Trichter, wobei der rezeptpflichtige Apothekenartikel von 6 Seiten gescannt wird, wobei der Data Matrix Code des Etiketts des rezeptpflichtigen Apothekenartikels erkannt wird und wobei der SecurPharm Code des rezeptpflichtigen Apothekenartikels auf dem rezeptpflichtigen Apothekenartikel erkannt wird;
ar) Vergleich des im gescannten Data Matrix Codes enthaltenen Etikettcodes und des gescannten SecurPharm Codes auf dem rezeptpflichtigen Apothekenartikel und der in Verfahrensschritt ap) gescannten Karton-Identifikationsnummer mit den digitalen Daten, die dem digitalen Kommissionierauftrag zugeordnet sind;
as) Wiederholung der Verfahrensschritte aq) bis ar) für alle in Verfahrensschritt am) auf die Fördertechnik abgegebenen rezeptpflichtigen Apothekenartikel, wobei alle rezeptpflichtigen Apothekenartikel in den gleichen Trichter abgegeben werden;
at) Abgabe des Haufens der nichtrezeptpflichtigen Apothekenartikel und/oder Drogerieartikel von der Fördertechnik in den Trichter, wobei der Haufen der nichtrezeptpflichtigen Apothekenartikel und/oder Drogerieartikel von 6 Seiten gescannt wird, wobei ein oder mehrere Identifikationscodes von Herstellern von nichtrezeptpflichtigen Apothekenartikel und/oder Drogerieartikel erkannt werden können;
au) Vergleich jedes Identifikationscodes eines Herstellers von einem nichtrezeptpflichtigen Apothekenartikel und/oder Drogerieartikel, der im Verfahrensschritt at) erkannt wird mit den Identifikationscodes der Hersteller, die im digitalen Kommissionierauftrag hinterlegt sind;
av) Abgabe der im Trichter gesammelten rezeptpflichtigen Apothekenartikel und nichtrezeptpflichtigen Apothekenartikel und/oder Drogerieartikel in den bereitgestellten Packkarton;
aw)Verschließen und Freigabe des Packkartons zur Versendung und Abmeldung der SecurPharm Codes der kommissionierten rezeptpflichtigen Apothekenartikel beim EU-Hub Netzwerk, wenn im Verfahrensschritt ar) für alle zu kommissionierenden rezeptpflichtigen Apothekenartikel festgestellt wurde, dass der Etikettcode und der SecurPharm Codes mit einem Etikettcode und einem SecurPharm Code übereinstimmen, die im digitalen Kommissionierauftrag hinterlegt sind und die gescannte Karton-Identifikationsnummer mit der Karton-Identifikationsnummer übereinstimmt, die im digitalen Kommissionierauftrages hinterlegt ist und wenn alle im Verfahrensschritt au) verglichenen Identifikationscodes von Herstellern im digitalen Kommissionierauftrag hinterlegt sind;
   oder Transport des Packkartons zu einer Fehlerkontrolle, wenn im Verfahrensschritt ar) für zumindest einen zu kommissionierenden rezeptpflichtigen Apothekenartikel festgestellt wurde, dass der Etikettcode oder der SecurPharm Code nicht mit einem Etikettcode oder einem SecurPharm Code übereinstimmen, die im digitalen Kommissionierauftrag hinterlegt sind; oder ein im Verfahrensschritt au) verglichener Identifikationscode eines Herstellers nicht im digitalen Kommissionierauftrag hinterlegt ist; oder Transport des Packkartons zu einer Fehlerkontrolle, wenn die gescannte Karton-Identifikationsnummer nicht mit der Karton-Identifikationsnummer übereinstimmt, die im digitalen Kommissionierauftrag hinterlegt ist.

Sämtliche nichtrezeptpflichtigen Apothekenartikel und/oder sonstigen Drogerieartikel, die mit dem digitalen Kommissionierauftrag verknüpft sind, werden erfindungsgemäß gemeinsam in einer weiteren leeren Pufferschale bereitgestellt. Durch das erfindungsgemäße Verfahren können dadurch nicht nur rezeptpflichtige Apothekenartikel, die auf einem analogen oder digitalen Rezept gelistet sind, kommissioniert werden, sondern auch weitere nichtrezeptpflichtige Apothekenartikel und/oder Drogerieartikel, die von einem Kunden gewünscht werden.

Ist eine Pufferschale mit nichtrezeptpflichtigen Apothekenartikeln und/oder Drogerieartikeln vorhanden, so wird der Inhalt dieser Pufferschale auf die gleiche Fördertechnik ausgegeben, wie der Inhalt der Pufferschalen mit rezeptpflichtigen Apothekenartikeln dieses digitalen Kommissionierauftrages. Die nichtrezeptpflichtigen Apothekenartikel und/oder Drogerieartikel werden aus der Pufferschale auf die Fördertechnik abgegeben, nachdem alle Pufferschalen mit rezeptpflichtigen Apothekenartikeln auf die Fördertechnik geleert worden sind. Ist mehr als ein nichtrezeptpflichtiger Apothekenartikel und/oder Drogerieartikel in der Pufferschale, bilden diese auf der Fördertechnik einen Haufen und werden als Haufen durch die Fördertechnik zur gleichen Packstation transportiert wie die rezeptpflichtigen Apothekenartikel des digitalen Kommissionierauftrages.

Nachdem alle rezeptpflichtigen Apothekenartikel von der Fördertechnik in den Trichter abgegeben wurden, bleibt der Trichter geschlossen und die auf der Fördertechnik transportierten nichtrezeptpflichtigen Apothekenartikel und/oder Drogerieartikel werden in den gleichen Trichter abgegeben, in dem sich bereits die rezeptpflichtigen Apothekenartikel des digitalen Kommissionierauftrages befinden. Bei der Abgabe der nichtrezeptpflichtigen Apothekenartikel und/oder Drogerieartikel in den Trichter werden diese, mit der für das Scannen der rezeptpflichtigen Apothekenartikel verwendeten Vorrichtung von 6 Seiten gescannt. Die nichtrezeptpflichtigen Apothekenartikel und/oder Drogerieartikel fallen als Haufen von der Fördereinrichtung in den Trichter, dabei werden von dem gesamten Haufen Bilder aufgenommen, wobei in einer Ausführungsform von einem oder mehreren nichtrezeptpflichtigen Apothekenartikel und/oder Drogerieartikeln mindestens ein Identifikationscode des Herstellers auf den Bildern erkannt und ausgelesen wird. Der mindestens eine gescannte Identifikationscode des Herstellers eines nichtrezeptpflichtigen Apothekenartikels und/oder eines Drogerieartikels wird dann mit den digitalen Daten, die dem digitalen Kommissionierauftrag zugeordnet sind, verglichen.

Sind alle mit dem digitalen Kommissionierauftrag verknüpften rezeptpflichtigen Apothekenartikel, nichtrezeptpflichtigen Apothekenartikel und/oder Drogerieartikel in dem Trichter, wird der Trichter geöffnet und sämtliche im Trichter gesammelten Artikel fallen in den Packkarton, der unter dem Trichter bereitgestellt ist.

Wenn bei der Abgabe der rezeptpflichtigen Apothekenartikel oder der nichtrezeptpflichtigen Apothekenartikel und/oder der Drogerieartikel von der vorherigen Fördertechnik in den Trichter eine Abweichung zwischen den gescannten Daten und den Daten des digitalen Kommissionierauftrages erkannt wurde, bzw. wenn eines der folgenden Ereignisse eintritt:
- zumindest ein vom Etikett eines rezeptpflichtigen Apothekenartikels gescannter Identifikationscode stimmt nicht mit dem im digitalen Kommissionierauftrag hinterlegten Identifikationscode des rezeptpflichtigen Apothekenartikels überein und/oder
- zumindest ein vom Etikett eines rezeptpflichtigen Apothekenartikels gescannter Identifikationscode stimmt nicht mit dem Identifikationscode überein, der direkt auf dem rezeptpflichtigen Apothekenartikel und/oder dessen Umverpackung aufgebracht ist und eingescannt wurde; und/oder
- die gescannte Karton-Identifikationsnummer stimmt nicht mit der Karton-Identifikationsnummer überein, die im digitalen Kommissionierauftrag hinterlegt ist; und/oder
- es kann vom Etikett eines rezeptpflichtigen Apothekenartikels keine Identifikationscode ausgelesen werde, z.B. weil das Etikett nicht richtig gescannt werden konnte; und/oder
- es kann keine Identifikationscode ausgelesen werden, der direkt auf dem rezeptpflichtigen Apothekenartikel und/oder dessen Umverpackung aufgebracht ist, z.B. weil dieser nicht richtig gescannt wurde; und/oder
- zumindest ein Identifikationscode des Herstellers eines nichtrezeptpflichtigen Apothekenartikels oder eines Drogerieartikels beim Scannen erkannt wurde und dieser Identifikationscode des Herstellers nicht im digitalen Kommissionierauftrag umfasst ist;
wird der Packkarton über die Fördereinrichtung einer Kontrolle übergeben zur Überprüfung übergeben.

Wird keine der genannten Abweichungen festgellt, wird der Packkarton verschlossen und zum Versand übergeben.

Bei der Abgabe der nichtrezeptpflichtigen Apothekenartikel und/oder Drogerieartikeln als Haufen in den Trichter muss erfindungsgemäß nicht zwangsläufig ein Identifikationscode eines Herstellers auf einem der nichtrezeptpflichtigen Apothekenartikel oder Drogerieartikel erkannt werden. Der 6-Seiten Scan dient in diesem Fall als stichprobenhafte Überprüfung des Kommissioniervorganges.

### Wägung

In einer Ausführungsform der vorliegenden Erfindung findet eine Überprüfung des kommissionierten Packkartons vor dem Verschließen und dem Versand über eine Gewichtskontrolle statt.

Hierfür ist für jeden rezeptpflichtigen Apothekenartikel und für jeden nichtrezeptpflichtigen Apothekenartikel und/oder Drogerieartikel dessen Gewicht bekannt. Diese Information kann zum Beispiel durch eine Wägung vor der Einlagerung in einem Warenlager ermittelt werden.

In dieser Ausführungsform werden die Packkartons vor dem Verschließen und dem Versand im Verfahrensschritt s) oder aw) zunächst einer Gewichtskontrolle übergeben.

Der Packkarton wird zu einer Waage transportiert und gewogen. Bevorzugt ist die Waage in die Fördertechnik integriert. Besonders bevorzugt ist die weitere Fördertechnik ein Förderband, in das eine Waage integriert ist.

Erfindungsgemäß wird ein Soll-Gewicht des Packkartons aus den bekannten Gewichten der kommissionierten rezeptpflichtigen Apothekenartikel und nichtrezeptpflichtigen Apothekenartikel und/oder Drogerieartikel berechnet. Das Leergewicht des Packkartons wurde dabei vor dem Befüllen mit den rezeptpflichtigen Apothekenartikel und nichtrezeptpflichtigen Apothekenartikel und/oder Drogerieartikel ermittelt und kann damit berücksichtigt werden. Bei der Wägung des Packkartons auf der Waage wird ein Ist-Gewicht bestimmt. Das Ist-Gewicht wird anschließend mit dem Soll-Gewicht verglichen. Wird zwischen Ist-Gewicht und Soll-Gewicht eine Abweichung festgestellt, die innerhalb eines vorgegebenen Sollbereichs liegt, so wird der Packkarton verschlossen und zur Versendung freigegeben. Wird jedoch zwischen dem Ist-Gewicht und Soll-Gewicht eine Abweichung festgestellt, die außerhalb eines vorgegebenen Toleranzbereiches liegt, wird der Packkarton über die Fördertechnik einer Kontrolle zugeführt. Der Toleranzbereich hängt dabei vom Gewicht des leichtesten Artikels im Packkarton ab. Die folgende Tabelle listet die Gewichtsgrenzen und die zugehörige Grenze des Toleranzbereichs auf.

| Gewicht | Grenzen des Toleranzbereichs |
|---|---|
| < 10 g | 20% des Artikelgewichtes |
| < 20 g | 50% des Artikelgewichtes |
| ≥ 20g | 70% des Artikelgewichtes |

Das bedeutet, wiegt der leichteste Artikel im Packkarton unter 10 g, werden Abweichungen des Ist-Gewichtes zum Soll-Gewicht bis zu 20 % dieses Artikels akzeptiert. Wiegt der leichteste Artikel im Packkarton hingegen zwischen 10 g und 20 g, werden Abweichungen des Ist-Gewichtes zum Soll-Gewicht bis zu 50 % dieses Artikels akzeptiert. Entsprechend Abweichungen des Ist-Gewichtes zum Soll-Gewicht bis zu 70 % eines Artikels akzeptiert, wenn der leichteste Artikel im Packkarton 20 g oder mehr wiegt.

Durch die Wägung kann geprüft werden, ob alle rezeptpflichtigen Apothekenartikel und nichtrezeptpflichtigen Apothekenartikel und/oder Drogerieartikel in den Packkarton kommissioniert worden sind, die laut digitalen Kommissionierauftrag kommissioniert werden sollten.

Liegt die Abweichung im Toleranzbereich, wird der Packkarton verschlossen und zum Versand freigegeben. Liegt die Abweichung jedoch außerhalb des Toleranzbereiches, wird der Packkarton einer Kontrolle zugeführt.

Das erfindungsgemäße Verfahren weist in dieser Ausführungsform daher zusätzlich die folgenden Schritte auf:
- Wiegen des Packkartons vor dem Verschließen zum Versand;
- Berechnen eines Soll-Gewichts des Packkartons aus den bekannten Gewichten der kommissionierten rezeptpflichtigen Apothekenartikel;
- Vergleich des Ist-Gewichts des Packkartons mit dem Soll-Gewicht des Packkartons;
- Verschließen und Freigabe des Packkartons zur Versendung, wenn eine Abweichung von Soll- und Ist-Gewicht innerhalb eines vorgegebenen Toleranzbereichs festgestellt wird oder Ausgabe des Packkartons auf eine Fördertechnik für eine Kontrolle, wenn eine Abweichung von Soll- und Ist-Gewicht außerhalb eines vorgegebenen Toleranzbereichs festgestellt wird.

### Kontrolle

Erfindungsgemäß werden die Packkartons deren Ist-Gewicht und Soll-Gewicht eine Abweichung voneinander aufweisen, die außerhalb des Toleranzbereiches liegt, zu einer Kontrolle befördert. Auch Packkartons, bei denen beim Befüllen des Packkartons aus dem Trichter Abweichung zwischen den gescannten Daten und den Daten des digitalen Kommissionierauftrages erkannt wurden, werden zu der bereits erwähnten Kontrolle befördert. Darüber hinaus werden die Packkartons zur Kontrolle befördert, wenn eine der bei der Kommissionierung verwendeten Maschinen (Fördertechnik, Warenlager, Handhabungsgerät usw.) einen Fehler meldet. Bei der Kontrolle werden erfindungsgemäß die folgenden Verfahrensschritte durchlaufen:
I. Transport des Packkartons zu einer Kontrollstation;
II. Scannen der Karton-Identifikationsnummer und Vergleich dieser mit den mit dem digitalen Kommissionierauftrag verknüpften digitalen Daten;
III. Auskippen des Inhalts des Packkartons in einen Behälter;
IV. Aufnahme eines rezeptpflichtigen Apothekenartikels oder eines nichtrezeptpflichtigen Apothekenartikels oder eines Drogerieartikels aus dem Behälter durch einen Roboter und Einscannen des Apothekenartikels von mindestens 6 Seiten, wobei bei rezeptpflichtigen Apothekenartikeln der Data Matrix Code des Etikettes und der SecurPharm Code auf dem rezeptpflichtigen Apothekenartikel gescannt wird und bei nichtrezeptpflichtigen Apothekenartikeln oder Drogerieartikeln der mindestens eine Identifikationscode des Herstellers auf dem nichtrezeptpflichtigen Apothekenartikel oder Drogerieartikel gescannt wird;
V. Vergleich des Etikettcodes und des SecurPharm Codes auf dem rezeptpflichtigen Apothekenartikel mit den digitalen Daten, die dem digitalen Kommissionierauftrag zugeordnet sind; oder Vergleich des mindestens einen gescannten Identifikationscodes des Herstellers mit den digitalen Daten, die dem digitalen Kommissionierauftrag zugeordnet sind für einen nichtrezeptpflichtigen Apothekenartikel oder einen Drogerieartikel;
VI. Ablegen des etikettierten rezeptpflichtigen Apothekenartikels oder des rezeptfreien Apothekenartikels oder des Drogerieartikels in dem Packkarton, wenn in Schritt V. und in Schritt II. die gescannten Daten mit den digitalen Daten, die dem digitalen Kommissionierauftrag zugeordnet sind, übereinstimmen; oder Auswurf in eine Fehlerschale wenn in Schritt V. oder in Schritt II. die gescannten Daten nicht mit den digitalen Daten des digitalen Kommissionierauftrages übereinstimmen;
VII. Wiederholen der Schritte IV. bis VI. für alle in Verfahrensschritt III. ausgekippten rezeptpflichtigen Apothekenartikel, nichtrezeptpflichtigen Apothekenartikel und Drogerieartikel;
VIII. Transport des Packkartons auf einer Fördertechnik zu einer Waage und wiegen des Packkartons, wenn im Verfahrensschritt VI. kein Apothekenartikel und/oder Drogerieartikel in eine Fehlerschale ausgeworfen wurde, wobei das ermittelte Gewicht das Ist-Gewicht des Packkartons ist;
IX. Berechnen des Soll-Gewichtes des Packkartons und Vergleich des Ist-Gewichts des Packkartons mit dem Soll-Gewicht des Packkartons;
X. Verschließen und Freigabe des Packkartons zur Versendung, wenn in Schritt IX. eine Abweichung von Soll- und Ist-Gewicht innerhalb eines vorgegebenen Toleranzbereichs festgestellt wird oder Ausgabe des Packkartons auf eine Fördertechnik für eine weitere Kontrolle, wenn in Schritt IX. eine Abweichung von Soll- und Ist-Gewicht außerhalb eines vorgegebenen Toleranzbereichs festgestellt wird.

Als Fördertechnik wird bevorzugt ein Rollenförderer genutzt. Im Verfahrensschritt IV. wird ein rezeptpflichtiger Apothekenartikel oder ein nichtrezeptpflichtiger Apothekenartikel oder ein Drogerieartikel mit einem Roboter aus einem Behälter aufgenommen. Hierfür kann beispielsweise ein Schwenkarmroboter eingesetzt werden. Das Scannen des aufgenommenen rezeptpflichtigen Apothekenartikels oder nichtrezeptpflichtigen Apothekenartikels oder Drogerieartikels kann beispielsweise durch mindestens 4 Digitalkameras durchgeführt werden, die an dem Schwenkarmroboter befestigt sind.

In einer weiteren Ausführungsform werden die Artikel nach dem Auskippen des Packkartons in den Behälter, aus diesem Entnommen und vereinzelt auf eine Fördertechnik gelegt.

Anschließend können die Artikel nach und nach in einen Trichter abgegeben werden, wobei sie von sechs Seiten gescannt werden. Ein geeigneter Trichter mit einer geeigneten Anordnung zum Scannen von sechs Seiten wurde bereits beschrieben und kommt hier bevorzugt genauso zum Einsatz.

Wird im Verfahrensschritt X. eine Abweichung von Soll- und Ist-Gewicht außerhalb eines vorgegebenen Toleranzbereichs festgestellt, wird der Packkarton zu einer manuellen Kontrolle durch einen Nutzer weitergeleitet. Geeignete Toleranzbereiche wurden bereits beschrieben und finden hier ebenso Anwendung.

In einer Ausführungsform der vorliegenden Erfindung werden im Verfahrensschritt v) nicht nur die Packkartons an eine Kontrolle übergeben, bei denen Soll- und Ist-Gewicht außerhalb eines vorgegebenen Toleranzbereichs voneinander abweichen, sondern zusätzlich ein Teil der kommissionierten Packkartons, für die keine Abweichung außerhalb eines vorgegebenen Toleranzbereichs festgestellt wurde. Diese Packkartons werden auf die Fördertechnik abgegeben und damit einer zusätzlichen Kontrolle zugeführt. Die Kontrolle läuft nach den bereits beschriebenen Verfahrensschritten I. bis X. ab. Auf diese Weise wird eine zusätzliche Qualitätskontrolle ermöglicht, die je nach Bedarf eingesetzt werden kann und deren Umfang an die Bedürfnisse eines Nutzers angepasst werden kann. Von einer Stichprobenkontrolle bis zur Kontrolle sämtlicher Packkartons ist erfindungsgemäß alles umsetzbar. In einer Ausführungsform der vorliegenden Erfindung werden zwischen 0% und 100% der kommissionierten Packkartons, bevorzugt werden 0% bis 25% der kommissionierten Packkartons, besonders bevorzugt werden 0% bis 5 % der kommissionierten Packkartons nach dem Verfahrensschritt s) oder aw) auf die Fördertechnik für eine zusätzliche Kontrolle ausgegeben. Dabei sind die ausgegebenen Packkartons für den Versand oder die Weiterbearbeitung vorgesehen und wiesen keine fehlerhafte Kommissionierung auf.

Mit dem erfindungsgemäßen Verfahren kann prinzipiell jede Art von rezeptpflichtigen Apothekenartikeln und nichtrezeptpflichtigen Apothekenartikeln und/oder Drogerieartikeln kommissioniert werden. Das heißt, sowohl Tablettenförmige als auch flüssige Artikel in Flaschenabfüllungen können das Verfahren durchlaufen. In einer besonders bevorzugten Ausführungsform ist der rezeptpflichtige Apothekenartikel quaderförmig. Quaderförmig kann dabei der Apothekenartikel an sich sein oder aber dessen Umverpackung, in der sich der Apothekenartikel befindet und in welchem dieser an einen Kunden abgegeben wird. Quaderförmige Umverpackungen haben den Vorteil, dass sie besonders geeignete Flächen zum Anbringen eines Etiketts aufweisen. Rezeptpflichtige Apothekenartikel, die in Flaschen abgefüllt sind, werden häufig auch in einer Umverpackung abgegeben, die meist quaderförmig ist.

In einer Ausführungsform der vorliegenden Erfindung, wird in Verfahrensschritt o) und/oder in Verfahrensschritt aq) oder at) weiterhin ein Foto oder mehrere Fotos von den zu kommissionierenden Apothekenartikeln im Trichter aufgenommen und/oder in Verfahrensschritt r) und/oder in Verfahrensschritt av) ein Foto oder mehrere Fotos vom Inhalt des Packkartons aufgenommen werden. Besonders bevorzugt wird ein oder werden mehrere digitale Fotos aufgenommen. Das Foto oder die Fotos werden bevorzugt mit dem digitalen Kommissionierauftrag verknüpft und gespeichert. Vorteilhafterweise kann das Foto oder können die Fotos zusätzlich dazu dienen, nachzuweisen, welche Artikel in den Packkarton kommissioniert wurden. Durch das Abspeichern des Fotos oder der Fotos ist ein optischer Nachweis des Kommissioniervorgangs auch nach Abschluss der Kommissionierung vorhanden. In einer weiteren Ausführungsform der vorliegenden Erfindung kann ein Foto oder können mehrere Fotos auch vor dem Verschließen des Packkartons zum Versenden aufgenommen werden. Auch dieses Foto oder diese Fotos dienen dem Nachweis der ordnungsgemäßen Kommissionierung und können verknüpft mit dem digitalen Kommissionierauftrag gespeichert werden.

### Einsortieren/Einlagern

In einer weiteren Ausführungsform der vorliegenden Erfindung umfasst das erfindungsgemäße Verfahren weiterhin das Sortieren von rezeptpflichtigen Apothekenartikeln in ein Warenlager. Der Schritt des Sortierens ist dem Kommissionieren vorangestellt und ermöglicht es rezeptpflichtige Apothekenartikel, die sortiert oder auch unsortiert zur Verfügung gestellt werden in ein Warenlager zu sortieren.

Das Verfahren weist dazu weiterhin folgende Verfahrensschritte auf:
- Bereitstellen einer Vielzahl von rezeptpflichtigen Apothekenartikeln;
- Vereinzeln der Vielzahl von rezeptpflichtigen Apothekenartikeln auf einer Fördertechnik, so dass jeder der rezeptpflichtigen Apothekenartikel mindestens einen vorgegebenen Abstand zu einem weiteren rezeptpflichtigen Apothekenartikel aufweist;

- Einscannen jedes der bereitgestellten rezeptpflichtigen Apothekenartikel von 6 Seiten;
- Erkennung des SecurPharm Codes auf jedem der eingescannten rezeptpflichtigen Apothekenartikel und von dessen Position auf jedem der eingescannten rezeptpflichtigen Apothekenartikel;
- Abspeichern des einen oder mehrerer Identifikationscodes eines Herstellers sowie der Position des SecurPharm Codes auf einem rezeptpflichtigen Apothekenartikel für den jeweils eingescannten rezeptpflichtigen Apothekenartikel und optional Erstellen eines Etikettcodes für jeden eingescannten rezeptpflichtigen Apothekenartikel;
- Einlagern der rezeptpflichtigen Apothekenartikel in einem Warenlager, bei denen sich der SecurPharm Code nicht auf der Oberseite des rezeptpflichtigen Apothekenartikels befindet;
- Ausgabe des rezeptpflichtigen Apothekenartikels in eine Fehlerschale, bei denen sich der SecurPharm Code auf der Oberseite des rezeptpflichtigen Apothekenartikels befindet.

Zunächst wird eine Vielzahl von sortierten und/oder unsortierten rezeptpflichtige Apothekenartikel bereitgestellt, etwa durch eine Warenlieferung in Form eines Paketes. Sortierte rezeptpflichtige Apothekenartikel sind Artikel die zu einem Typ von rezeptpflichtigen Apothekenartikel gehören. Beispielsweise ein Karton der mit einem Typ von rezeptpflichtigen Apothekenartikeln gefüllt ist. Unsortierte rezeptpflichtige Apothekenartikel hingegen sind beispielsweise Kartons, die mit verschiedenen Typen von rezeptpflichtigen Apothekenartikeln gefüllt sind. Der Anzahl an bereitgestellten rezeptpflichtigen Apothekenartikel ist keine Grenze gesetzt. Mit dem erfindungsgemäßen Verfahren können bis zu 500 rezeptpflichtige Apothekenartikel pro Stunde in ein Warenlager sortiert werden.

Werden die rezeptpflichtigen Apothekenartikel in Kartons angeliefert, so werden diese geöffnet und einer Vereinzelungsmaschine übergeben. Die Vereinzelungsmaschine separiert, d.h. vereinzelt die Vielzahl von rezeptpflichtigen Apothekenartikel auf einer Fördertechnik.

Besonders bevorzugt ist diese Fördertechnik ein Förderband. Geeignet Vereinzelungsmaschinen sind dem Fachmann aus dem Stand der Technik bereits bekannt.

Durch die Vereinzelungsmaschine kommen die rezeptpflichtigen Apothekenartikel beabstandet zueinander auf der Fördertechnik zu liegen. Beabstandet bedeutet, dass zwischen jedem rezeptpflichtigen Apothekenartikel auf der Fördertechnik und einem darauffolgenden rezeptpflichtigen Apothekenartikel ein Mindestabstand besteht. In einer Ausführungsform der Erfindung beträgt der Abstand zwischen eine rezeptpflichtigen Apothekenartikel und einem darauffolgenden Apothekenartikel mindestens 20 cm, bevorzugt 30 cm.

Anschließend wird jeder Apothekenartikel von 6 Seiten gescannt. Hierfür wird bevorzugt eine Vorrichtung verwendet, bei der an einem Gestell mindestens 6 Digitalkameras befestigt sind. Jeder Apothekenartikel wird hierfür über eine Glasscheibe transportiert, so dass auch von unten Bilder aufgenommen werden können.

Beim Einscannen wird mindestens der SecurPharm Code auf jedem der eingescannten rezeptpflichtigen Apothekenartikel erkannt, sowie die dessen Position auf jedem der eingescannten rezeptpflichtigen Apothekenartikel. Diese Daten werden für den jeweils eingescannten rezeptpflichtigen Apothekenartikel abgespeichert und optional wird ein Etikettcode für jeden eingescannten rezeptpflichtigen Apothekenartikel erstellt. Der Etikettcode wird dann ebenfalls gespeichert. Die Daten können beispielsweise in einer geeigneten Datenbank hinterlegt werden.

Das Einscannen der einzelnen rezeptpflichtigen Apothekenartikel kann erfindungsgemäß nacheinander für jeden Artikel einzeln erfolgen, da jeder rezeptpflichtige Apothekenartikel beabstandet zu einem weiteren rezeptpflichtigen Apothekenartikel auf der Fördertechnik liegt. Hierdurch wird ausgeschlossen, dass von zwei oder noch mehr rezeptpflichtige Apothekenartikel zur gleichen Zeit durch die Digitalkameras Bilder aufgenommen werden.

Die rezeptpflichtigen Apothekenartikel werden in einem angeschlossenen Warenlager eingelagert. Eine Einlagerung wird jedoch nur unter der Voraussetzung vorgenommen, dass sich auf der Oberseite des rezeptpflichtigen Apothekenartikels nicht der SecurPharm Code des rezeptpflichtigen Apothekenartikels befindet. Als Oberseite wird dabei die Seite des rezeptpflichtigen Apothekenartikels verstanden, die sich gegenüber der Seite des rezeptpflichtigen Apothekenartikels befindet, die auf der Fördertechnik liegt. Damit kann sichergestellt werden, dass zumindest die Oberseite des rezeptpflichtigen Apothekenartikels zur Verfügung steht, um auf dieser erfindungsgemäß ein Etikett aufzubringen, so dass mindestens ein Identifikationscode des rezeptpflichtigen Apothekenartikels nicht verdeckt wird.

Die rezeptpflichtigen Apothekenartikel, auf deren Oberseite sich der einzige Identifikationscode des rezeptpflichtigen Apothekenartikels befindet, werden in eine Fehlerschale ausgegeben. Die Fehlerschale kann dann beispielsweise einer manuellen Überprüfung durch einen Nutzer zugeführt werden.

### Vorrichtung

Weiterhin stellt die vorliegende Erfindung eine Vorrichtung zur Durchführung des Verfahrens zur vollautomatischen Kommissionierung von rezeptpflichtigen und optional von nichtrezeptpflichtigen Apothekenartikeln aus einem Warenlager zur Verfügung. Die Vorrichtung weist erfindungsgemäß
- Mindestens eine Recheneinheit;
- Mindestens eine Etikettier Maschine;
- Mindestens eine Fördertechnik mit Anschluss an ein Warenlager;
- Einen Trichter mit einem Kamerasystem oder einem Barcodelesesystem, wobei das Kamerasystem oder das Barcodelesesystem mindestens 6 Kameras oder Barcodeleser aufweist;
- Mindestens eine zweite Fördertechnik;
- Mindestens einen Scanner;
- Optional Mindestens eine Waage;
- Optional mindestens einen Roboter mit einer Greifeinheit;
auf.

Alle Merkmale, die für das erfindungsgemäße Verfahren beschrieben wurden, treffen im gleichen Maße auf die erfindungsgemäße Vorrichtung zu und umgekehrt.

In einer besonders bevorzugten Ausführungsform der vorliegenden Erfindung ist die erfindungsgemäße Vorrichtung dazu eingerichtete, das erfindungsgemäße Verfahren durchzuführen.

Die Vorrichtung umfasst mindestens eine Recheneinheit. Als Recheneinheit ist beispielsweise ein PC geeignet.

Weiterhin umfasst die Vorrichtung mindestens einen Etikettierer. Der Etikettierer ist eine Vorrichtung mit einem Drucker, mit dem auf ein Blankoetikett Informationen gedruckt werden können. Der Etikettierer umfasst weiterhin eine Vorrichtung, mit der ein Etikett auf einen rezeptpflichtigen Apothekenartikel aufgebracht werden kann. Besonders bevorzugt werden selbstklebende Etiketten verwendet. Geeignete Etikettiermaschinen sind aus dem Stand der Technik bereits bekannt.

Die erfindungsgemäße Vorrichtung umfasst weiterhin mindestens eine Fördertechnik mit Anschluss an ein Warenlager. Die Fördertechnik ist besonders bevorzugt ein Förderband. Vom Warenlager wird ein rezeptpflichtiger Apothekenartikel nach dem Etikettieren in einer Pufferschale zur Verfügung gestellt. Die Pufferschale ist derart angeordnet, dass deren Inhalt auf die Fördertechnik abgegeben werden kann. Weiterhin ist die Fördertechnik so angeordnet, dass auch eine Pufferschale mit nichtrezeptpflichtigen Apothekenartikeln und/oder Drogerieartikeln auf die Fördertechnik geleert werden kann.

Am Ende der Fördertechnik befindet sich ein Trichter. Der Trichter weist am Trichterhals einen Boden auf, der geöffnet und geschlossen werden kann. Der Trichter weist in Kamerasystem oder ein Barcodelesesystem mit mindestens 6 Kameras oder Barcodelesern auf. Geeignete Kameras sind beispielsweise Digitalkameras. Geeignete Anzahlen und Anordnungen der Digitalkameras oder Barcodeleser wurden bereits beschrieben. Auf diese Ausführungen sei an dieser Stelle verwiesen.

Die erfindungsgemäße Vorrichtung weist mindestens eine zweite Fördertechnik auf. Auf der zweiten Fördertechnik wird bevorzugt der Packkarton transportiert. Die zweite Fördertechnik ist bevorzugt ein Rollenförderer. Die zweite Fördertechnik ist derart gestaltet, dass ein Packkarton vom Trichter zu einer Versandstation transportiert werden kann oder vom Trichter zu einer weiteren Kontrolle. Die weitere Kontrolle findet bevorzugt auf der zweiten Fördertechnik statt. Die zweite Fördertechnik kann hierfür eine geeignete Weiche aufweisen.

Weiterhin weist die erfindungsgemäße Vorrichtung einen Scanner auf, mit dem die Karton-Identifikationsnummer eingescannt werden kann. Der Scanner ist demnach derart angeordnet, dass das Einlesen der Karton-Identifikationsnummer ermöglicht wird, während sich der Karton auf der zweiten Fördertechnik befindet. Ein geeigneter Scanner ist beispielsweise eine Digitalkamera oder ein stationärer Barcodeleser.

Die Vorrichtung weist weiterhin mindestens eine Waage auf. In einer bevorzugten Ausführungsform ist, die mindestens eine Waage in der zweiten Fördertechnik integriert. Diese kann besonders bevorzugt eine Durchlaufwaage in einem Rollenförderer sein. In einer bevorzugten Ausführungsform weist die Vorrichtung eine zweite Waage auf, wobei die zweite Waage ebenfalls bevorzugt in Form einer Durchlaufwaage in einem Rollenfördere vorliegt. Die zweite Waage ermöglicht die Wägung bei der Kontrolle der gefüllten Packkartons.

Optional weist die Vorrichtung einen Roboter mit einem Greifarm, zum Beispiel einen Schwenkarm Roboter auf. Dieser wird genutzt, um bei einer Kontrolle sämtliche Artikel einzeln aufzunehmen und von 6 Seiten zu scannen. Diese Verfahrensschritte wurden bereits beschrieben.

In einer Ausführungsform ist das erfindungsgemäße Verfahren dazu geeignet rezeptpflichtige Apothekenartikel in ein Warenlager zu sortieren und zu kommissionieren. Daher weist die erfindungsgemäße Vorrichtung in einer Ausführungsform weiterhin
- Mindestens eine Vereinzelungsmaschine;
- Mindestens eine weitere Fördertechnik;
- Mindestens eine Station an der ein rezeptpflichtiger Apothekenartikel von 6 Seiten gescannt werden kann;
- Ein Warenlager;
auf.

Vereinzelungsmaschinen wie sie dem Fachmann aus dem Stand der Technik bekannt sind, sind für die vorliegende Erfindung geeignet.

Die Vorrichtung weist in dieser Ausführungsform mindestens eine weitere Fördertechnik auf. Die mindestens eine weitere Fördertechnik ist bevorzugt ein Förderband. Von der Vereinzelungsmaschine wird die Vielzahl an rezeptpflichtigen Apothekenartikeln auf die mindestens eine Fördertechnik vereinzelt ausgegeben und über die Fördertechnik dann zu der mindestens einen Station transportiert an der ein rezeptpflichtiger Apothekenartikel von 6 Seiten gescannt werden kann.

Die mindestens eine Station weist Kameras oder stationäre Barcodescanner auf, die derart angeordnet sind, dass jeder Artikel von 6 Seiten gescannt werden kann. Jeder Artikel wird dabei vereinzelt auf eine Glasscheibe gelegt oder über diese transportiert, so dass auch von der Unterseite des Artikels, die mit der Glasscheibe in Berührung ist ein Bild aufgenommen werden kann, bzw. diese gescannt werden kann.

Weiterhin weist die Vorrichtung ein Warenlager auf, in das jeder gescannte rezeptpflichtige Apothekenartikel eingelagert wird, bei denen sich auf der Oberseite des rezeptpflichtigen Apothekenartikels nicht der SecurPharm Code des jeweiligen rezeptpflichtigen Apothekenartikels befindet. Geeignete Warenlager sind beispielsweise Warenlager nach dem ABC-Lagersystem oder Warenlager nach dem Chaotischen Prinzip. Besonders bevorzugt ist das Warenlager ein KNAPP-Store der Firma KNAPP AG, welches nach dem Chaotischen Prinzip einlagert.

Im Folgenden wird die Erfindung weiterhin durch 3 Figuren und 4 Ausführungsbeispiele näher erläutert.
- Figur 1: stellt ein Ablaufschema einer Ausführungsform des erfindungsgemäßen Verfahrens dar;
- Figur 2: (A) bis (C) stellen eine Ausführungsform des Trichters mit Förderband, Digitalkameras und Packkarton aus verschiedenen Blickwinkeln dar;
- Figur 3: stellt einen rezeptpflichtigen Apothekenartikel mit einem Etikett dar.

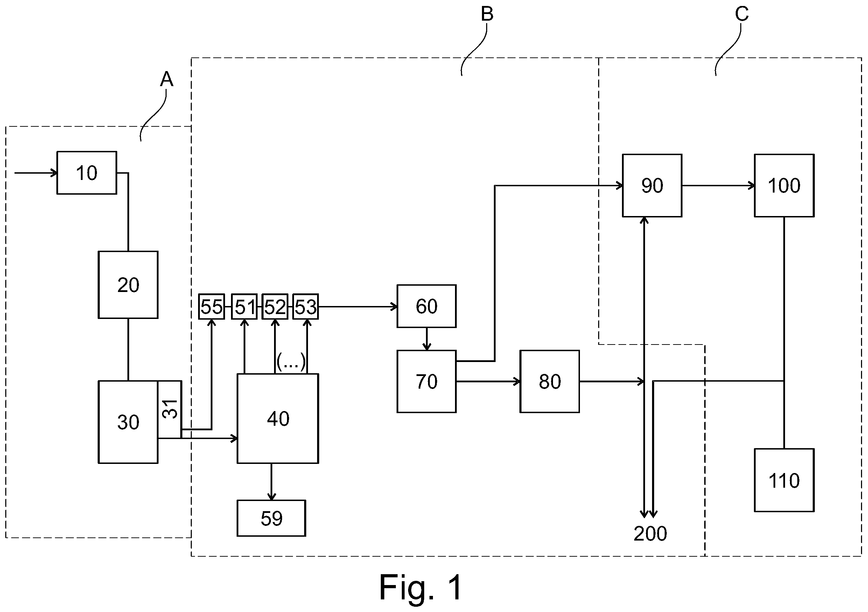

**Figur 1** stellt ein Ablaufschema einer Ausführungsform des erfindungsgemäßen Verfahrens dar in dem rezeptpflichtige Apothekenartikel sortiert (Abschnitt A) und kommissioniert (Abschnitt B) werden. Beim Sortieren der rezeptpflichtigen Apothekenartikel werden diese sortiert oder unsortiert bereitgestellt und durch einen Vereinzler 10 auf eine Fördertechnik vereinzelt ausgegeben. Jeder rezeptpflichtige Artikel wird durch die Fördertechnik zu einer Station 20 transportiert, an der jeder rezeptpflichtige Apothekenartikel einzeln von 6 Seiten gescannt wird. Beim Scannen wird mindestens ein Identifikationscode auf jedem rezeptpflichtigen Apothekenartikel erkannt und dessen Position auf dem jeweiligen rezeptpflichtigen Apothekenartikel. Erfindungsgemäß wird ein Produktcode erzeugt, der mindestens eine Identifikationsnummer des rezeptpflichtigen Apothekenartikels aufweist. Anschließend werden die rezeptpflichtigen Apothekenartikel in einem Warenlager 30 eingelagert, bei denen sich auf der Oberseite des rezeptpflichtigen Apothekenartikels nicht den SecurPharm Code des rezeptpflichtigen Apothekenartikels befindet.

Das erfindungsgemäße Verfahren ermöglicht vor allem das Kommissionieren von rezeptpflichtigen Apothekenartikeln wie in einer Ausführungsform in Abschnitt B der Figur 1 dargestellt. Hierfür wird ein elektronisches Rezept bereitgestellt und ein digitaler Kommissionierauftrag erstellt. Das elektronische Rezept wird eingelesen und die Daten des Rezeptes werden mit dem digitalen Kommissionierauftrag verknüpft. Optional können noch mehr elektronische Rezepte bereitgestellt werden, wobei deren Daten ebenfalls mit dem digitalen Kommissionierauftrag verknüpft werden. Erfindungsgemäß wird ein rezeptpflichtiger Apothekenartikel aus einem Warenlager 30 bereitgestellt und dessen Etikettcode dem digitalen Kommissionierauftrag zugeordnet. In einem Etikettierer 40 wird ein Etikett erstellt, auf welchem personenbezogene Daten des Rezeptempfängers, Angaben zur Dosierung des bereitgestellten rezeptpflichtigen Apothekenartikels, ein Data Matrix-Code aufweisend den Etikettcode des bereitgestellten rezeptpflichtigen Apothekenartikels und optional weiterer Hinweise des Arztes, die auf einem elektronischen Rezept bezüglich des rezeptpflichtigen Apothekenartikels enthalten sind, gedruckt sind. Das bedruckte Etikett wird auf dem rezeptpflichtigen Apothekenartikel derart aufgebracht, dass das Etikett den SecurPharm Code des rezeptpflichtigen Apothekenartikels nicht verdeckt. Anschließend wird der Data Matrix Codes des Etiketts eingescannt und er darin enthaltene Etikettcode ausgelesen. Der ausgelesene Etikettcode wird mit den digitalen Daten verglichen, die mit dem digitalen Kommissionierauftrag verknüpft sind. Wenn bei diesem Vergleich keine Abweichungen festgestellt werden, dann wird der rezeptpflichtige Apothekenartikel in einer leeren Pufferschale 51 abgelegt. Wird jedoch eine Abweichung festgestellt, wird der rezeptpflichtige Apothekenartikel in einer Fehlerschale 59 abgelegt, in gleicher Weise wird verfahren, wenn das Etikettieren nicht möglich sein sollte. Keine Abweichung bedeutet, dass der gescannte Etikettcode mit einem Etikettcode übereinstimmt, der in den digitalen Daten des digitalen Kommissionierauftrages hinterlegt ist.

Anschließend wird ein nächster rezeptpflichtiger Apothekenartikel aus einem Warenlager 30 bereitgestellt, der ebenfalls wie bereits beschrieben in einem Etikettierer 40 mit einem Etikett versehen wird. Der rezeptpflichtige Artikel wird nach dem Etikettieren, Einscannen des Etiketts, Auslesen des Etikettcodes und dem Vergleich mit den digitalen Daten, die mit dem digitalen Kommissionierauftrag verknüpft sind, entweder in eine weitere leere Pufferschale 52 ausgegeben oder in eine Fehlerschale 59. Je nach Ergebnis des Vergleichs des gescannten Etikettcodes und der Daten die mit dem digitalen Kommissionierauftrag verknüpft sind.

Sind alle rezeptpflichtigen Apothekenartikel, die mit dem digitalen Kommissionierauftrag verknüpft sind, etikettiert und in Pufferschalen abgelegt, werden die Pufferschalen 51, 52, 53 nacheinander auf eine Fördertechnik geleert. Dabei kommen die rezeptpflichtigen Apothekenartikel beabstandet zueinander oder zu einem weiteren Artikel auf der Fördertechnik zu liegen.

Die rezeptpflichtigen Apothekenartikel werden über die Fördertechnik zu einem Trichter 60 transportiert. Unterhalb des Trichters 60 wird ein Packkarton 70 bereitgestellt, der mit einem Etikett mit einer Karton-Identifikationsnummer versehen ist. Die Karton-Identifikationsnummer wird verfahrensgemäß vom Etikett des Packkartons gescannt. Am Boden des Trichters 60 befindet sich eine Öffnung, die geöffnet und verschlossen werden kann.

Eines der auf der Fördertechnik transportierten rezeptpflichtigen Apothekenartikel wird in den Trichter abgegeben. Dabei wird der rezeptpflichtige Apothekenartikel von 6 Seiten gescannt, wobei der Data Matrix Code des Etiketts (Etikettcode) des rezeptpflichtigen Apothekenartikels erkannt wird und wobei der SecurPharm Code des rezeptpflichtigen Apothekenartikels auf dem rezeptpflichtigen Apothekenartikel erkannt wird. Der gescannte Etikettcode und der gescannten SecurPharm Code auf dem rezeptpflichtigen Apothekenartikel und die gescannte Karton-Identifikationsnummer werden mit den digitalen Daten, die dem digitalen Kommissionierauftrag zugeordnet sind, verglichen. Nacheinander wird dies für sämtliche rezeptpflichtigen Apothekenartikel durchgeführt, die sich auf der Fördertechnik befinden und die zu dem digitalen Kommissionierauftrag gehören. Die rezeptpflichtigen Apothekenartikel werden gemäß dem erfindungsgemäßen Verfahren in dem Trichter 60 gesammelt, dessen Boden solange geschlossen bleibt. Sind alle rezeptpflichtigen Apothekenartikel des digitalen Kommissionierauftrages im Trichter 60 wird dessen Boden geöffnet und die Artikel fallen vom Trichter in den Packkarton. Der Packkarton wird über eine Fördertechnik zu einer Waage 80 transportiert. Wenn keine Abweichung der bei der Abgabe der rezeptpflichtigen Apothekenartikel in den Trichter vom Etikett und von den rezeptpflichtigen Apothekenartikeln gescannten Daten, der Karton-Identifikationsnummer und den mit dem digitalen Kommissionierauftrag verknüpften Daten festgestellt wurden, wird der Packkarton auf der Waage 80 gewogen. Wurden Abweichungen festgestellt, wird der Packkarton einer Kontrollstation mit einem Roboter mit Greifeinheit 90 zugeführt.

Wird keine Abweichung festgestellt wird der Packkarton 70 auf der Waage 80 gewogen. Bei der Wägung ergibt sich ein Ist-Gewicht. Aus den bekannten Gewichten der kommissionierten rezeptpflichtigen Apothekenartikel wird erfindungsgemäß ein Soll-Gewicht berechnet, dass mit dem Ist-Gewicht verglichen wird. Der Packkarton 70 wird verschlossen und zur Versendung 200 freigegeben, wenn eine Abweichung von Soll- und Ist-Gewicht innerhalb eines vorgegebenen Sollbereichs liegt. Liegt die Abweichung von Soll- und Ist-Gewicht jedoch außerhalb des vorgegebenen Sollbereichs, wird der Packkarton einer Kontrollstation mit einem Roboter mit Greifeinheit 90 zugeführt.

In einer Ausführungsform der vorliegenden Erfindung werden zusätzlich zu rezeptpflichtigen Apothekenartikeln auch nichtrezeptpflichtige Apothekenartikel und/oder Drogerieartikel kommissioniert. Hierfür werden die nichtrezeptpflichtigen Apothekenartikel und/oder Drogerieartikel aus einem Warenlager 31 oder dem Warenlager 30 gesammelt in einer Pufferschale 55 zur Verfügung gestellt. Der Inhalt der Pufferschale 55 wird nach dem Entleeren der Pufferschalen 51, 52, 53 mit den rezeptpflichtigen Apothekenartikeln auf eine Fördertechnik entleert. Hierfür wird die gleiche Fördertechnik verwendet, wie für die Pufferschalen 51, 52, 53 mit den rezeptpflichtigen Apothekenartikeln. Die nichtrezeptpflichtigen Apothekenartikel und/oder Drogerieartikel werden beabstandet zu weiteren Artikeln als Haufen auf die Fördertechnik ausgegeben. Der gesamte Haufen wird in den Trichter 60 abgegeben, indem sich bereits die rezeptpflichtigen Apothekenartikel befinden.

Hierbei werden die nichtrezeptpflichtigen Apothekenartikel und/oder Drogerieartikel von 6 Seiten gescannt. Dabei wird mindestens ein Identifikationscode auf einem nichtrezeptpflichtigen Apothekenartikel und/oder einem Drogerieartikel erkannt. Der mindestens eine eingescannte Identifikationscode wird mit den digitalen Daten, die dem digitalen Kommissionierauftrag zugeordnet sind, verglichen. Die im Trichter gesammelten Artikel werden in den Packkarton 60 abgegeben. Wenn die eingescannten Daten der rezeptpflichtigen Apothekenartikel, die eingescannten Daten der nichtrezeptpflichtigen Apothekenartikel und/oder Drogerieartikel und der eingescannte Karton-Identifikationscode mit den digitalen Daten übereinstimmen, die diesbezüglich mit dem digitalen Kommissionierauftrag verknüpft sind, wird der Packkarton zu einer Waage transportiert und ein Ist-Gewicht gewogen. Unter Berücksichtigung der nichtrezeptpflichtigen Apothekenartikel und/oder Drogerieartikel wird ein Soll-Gewicht des Packkartons berechnet. Wie bereits beschrieben werden Ist- und Soll-Gewicht miteinander verglichen und die Abweichung mit einem vorgegebenen Sollbereich verglichen. Der Packkarton 70 wird verschlossen und zur Versendung 200 freigegeben, wenn eine Abweichung von Soll- und Ist-Gewicht innerhalb eines vorgegebenen Sollbereichs liegt. Liegt die Abweichung von Soll- und Ist-Gewicht jedoch außerhalb des vorgegebenen Sollbereichs, wird der Packkarton einer Kontrollstation mit einem Roboter mit Greifeinheit 90 zugeführt.

Der Ablauf der Kontrolle ist in Abschnitt C dargestellt. Für eine Kontrolle wird der Packkarton 70 zu einer Station 90 mit einem Roboter mit einem Greifarm über reine Fördertechnik befördert. In einer Ausführungsform der vorliegenden Erfindung wird der Inhalt des Packkartons auf eine Fördertechnik, bevorzugt in einen Behälter, ausgekippt. Die Karton-Identifikationsnummer wird vom Packkarton gescannt und mit den Daten, die mit dem digitalen Kommissionierauftrag verknüpft sind, verglichen. Anschließend wird jeder rezeptpflichtige Artikel von dem Roboter aufgenommen und von 6 Seiten gescannt. Bei rezeptpflichtigen Apothekenartikeln wird hierbei der Data Matrix Code des Etikettes und der SecurPharm Code auf dem rezeptpflichtigen Apothekenartikel gescannt und bei nichtrezeptpflichtigen Apothekenartikeln oder Drogerieartikeln der mindestens eine Identifikationscode auf dem nichtrezeptpflichtigen Apothekenartikel oder Drogerieartikel. Der im gescannten Data Matrix Code enthaltene Etikettcode und der gescannte SecurPharm Code auf einem rezeptpflichtigen Apothekenartikel werden mit den digitalen Daten, die dem digitalen Kommissionierauftrag zugeordnet sind, verglichen. Für einen nichtrezeptpflichtigen Apothekenartikel oder einen Drogerieartikel wird der mindestens eine gescannte Identifikationscode mit den digitalen Daten, die dem digitalen Kommissionierauftrag zugeordnet sind, verglichen.

Werden keine Abweichungen beim Vergleich festgestellt, wird der etikettierte rezeptpflichtige Apothekenartikel oder der rezeptfreie Apothekenartikel oder der Drogerieartikel in dem Packkarton abgelegt. Wird hingegen eine Abweichung festgestellt wird der der etikettierte rezeptpflichtige Apothekenartikel oder der rezeptfreie Apothekenartikel oder der Drogerieartikel in eine Fehlerschale ausgegeben.

Diese beschriebenen Verfahrensschritte werden für sämtliche Artikel, die aus dem Packkarton ausgekippt worden sind, wiederholt. Der Packkarton wird anschließend auf einer Fördertechnik zu einer Waage 100 transportiert und gewogen, wenn bei der Kontrollstation 90 kein Apothekenartikel und/oder Drogerieartikel in eine Fehlerschale ausgeworfen wurde. Das ermittelte Gewicht stellt das Ist-Gewicht des Packkartons dar. Das Ist-Gewicht des Packkartons wird mit dem Soll-Gewicht des Packkartons verglichen und wenn das Ist-Gewicht und das Soll-Gewicht nur innerhalb eines vorgegebenen Sollbereiches voneinander abweichen wird der Packkarton 70 verschlossen und zum Versand 200 freigegeben. Weichen Ist- Und Soll-Gewicht jedoch außerhalb des vorgegebenen Sollbereiches voneinander ab, wird der Packkarton 70 einer manuellen Kontrolle 110 übergeben.

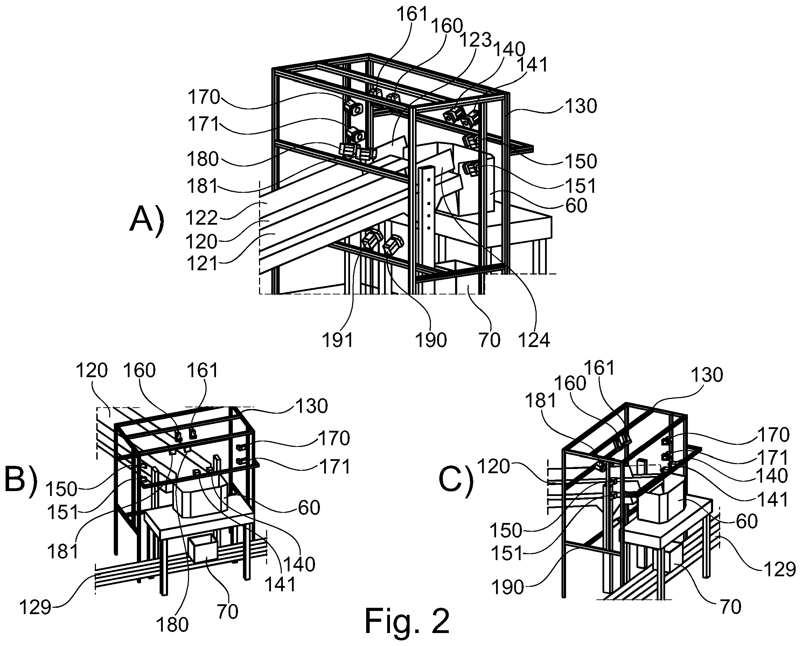

**Figur 2 (A) bis (C)** stellt eine Ausführungsform des Trichters 60 mit Förderband 120, Digitalkameras 140, 141, 150, 151, 160, 161, 170, 171, 180, 181 und Packkarton 70 aus verschiedenen Blickwinkeln dar.

Dabei sind an einem Rahmen 130 eine erste und eine zweite Digitalkamera 160, 161 oberhalb der Fördertechnik etwas beabstandet voneinander angebracht, eine dritte und eine vierte Digitalkamera 150, 151 rechts und eine fünfte und eine sechste Digitalkamera 170, 171 links der Fördertechnik positioniert. Eine siebte und eine achte Digitalkamera 180, 181 sind so positioniert, dass sie Bilder von dem rezeptpflichtigen Apothekenartikel aufnehmen können, nachdem dieser die siebte und achte Digitalkamera 180, 181 passiert hat. Eine neunte und zehnte Digitalkamera 140, 141 ist derart positioniert, dass sie Bilder von dem rezeptpflichtigen Apothekenartikel aufnehmen können, während dieser sich auf der Fördertechnik auf die neunte und zehnte Digitalkamera 140, 141 zubewegt. Eine elfte und eine zwölfte Digitalkamera 190, 191 sind derart positioniert, dass sie von unten Bilder von dem rezeptpflichtigen Apothekenartikel aufnehmen können, während dieser von der Fördertechnik 120 in den Trichter 60 fällt. Die 12 Digitalkameras sind damit an 6 verschiedenen Positionen angeordnet, so dass von 6 unterschiedlichen Seiten Bilder des rezeptpflichtigen Apothekenartikels aufgenommen werden können.

In der gezeigten Ausführungsform ist die Fördertechnik ein Förderband 120 mit einem Seitenschutz 121, 122, damit keiner der transportierten Artikel seitlich vom Förderband 120 fallen kann. Der Seitenschutz 121, 122 ist am Ende des Förderbandes 120, welches sich am Trichter 60 befindet, durchsichtig 123, 124 ausgebildet ist. Dies ist vorteilhafterweise innerhalb des Blickwinkels der Digitalkameras die rechts 150, 151 und links 170, 171 des Förderbandes 120 befinden der Fall. Auf diese Weise wird der Blickwinkel der Digitalkameras 150, 151, 170, 171 nicht durch den Seitenschutz 123, 124 beeinträchtigt.

Der Packkarton 70 wird auf dem Förderband 129 transportiert.

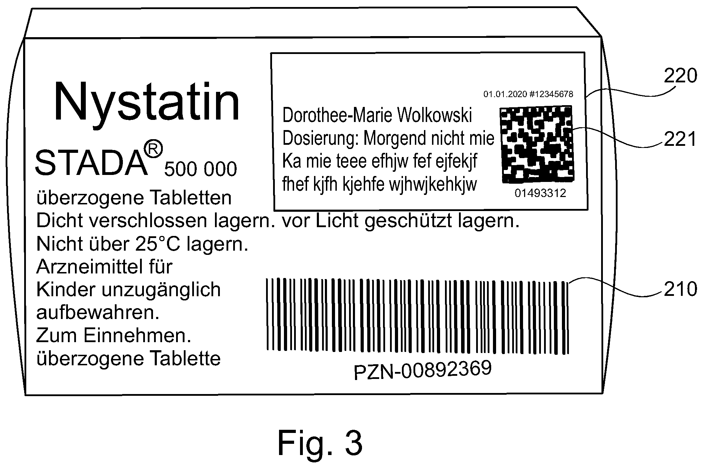

**Figur 3** stellt einen rezeptpflichtigen Apothekenartikel 210 mit einem Etikett 220 dar. Das Etikett weist personenbezogene Daten und einen Data Matrix Code 221 auf, wobei der Data Matrix Code 221 einen Etikettcode aufweist.

### Ausführungsbeispiel 1 - Kommissionieren von rezeptpflichtigen Apothekenartikeln

Es wurde ein digitales Rezept über eine App für einen Kunden eingereicht. Das digitale Rezept wurde eingelesen und ein digitaler Kommissionierauftrag wurde erstellt. Mit dem elektronischen Rezept wurden drei rezeptpflichtige Apothekenartikel verordnet, wobei für jeden rezeptpflichtigen Apothekenartikel eine Dosieranweisung seitens des Arztes vermerkt war. Dem digitalen Kommissionierauftrag wurden sämtliche Daten des elektronischen Rezeptes zugeordnet. Insbesondere folgende Informationen:
- Name und Adresse des Kunden;
- Rezeptpflichtige Apothekenartikel, die mit dem elektronischen Rezept verordnet wurden;
- Dosieranweisungen für jeden verordneten rezeptpflichtigen Apothekenartikel.

Der erste rezeptpflichtige Apothekenartikeln wurde aus einem Warenlager 30 zur Verfügung gestellt, mit dem Etikettierer 40 etikettiert, eingescannt und in einer leeren Pufferschale 51 abgelegt. Anschließend wurde der zweite rezeptpflichtige Apothekenartikeln aus dem Warenlager 30 zur Verfügung gestellt und durchlief die gleichen Verfahrensschritte wie zuvor der erste rezeptpflichtige Apothekenartikel. Der zweite rezeptpflichtige Apothekenartikel wurde anschließend in einer leeren Pufferschale 52 abgelegt. Anschließend wurde der dritte rezeptpflichtige Apothekenartikeln aus dem Warenlager 30 zur Verfügung gestellt und durchlief die gleichen Verfahrensschritte wie zuvor der erste und zweite rezeptpflichtige Apothekenartikel. Der dritte rezeptpflichtige Apothekenartikel wurde anschließend in einer leeren Pufferschale 53 abgelegt. Beim Vergleich der gescannten Daten mit den Daten, die mit dem digitalen Kommissionierauftrag verknüpft waren, wurden keine Abweichungen festgestellt. Die Pufferschalen 51, 52, 53 wurden zeitlich versetzt auf ein Förderband 120 geleert. Der erste rezeptpflichtige Apothekenartikel kam mit einem Anstand von 30 cm zum zweiten rezeptpflichtigen Apothekenartikel auf dem Förderband 120 zu liegen. Der zweite rezeptpflichtige Apothekenartikel kam mit einem Anstand von 30 cm zum dritten rezeptpflichtigen Apothekenartikel auf dem Förderband 120 zu liegen.

Die drei rezeptpflichtigen Apothekenartikel wurden mittels des Förderbandes 120 zu einem Trichter 60 transportiert. Unterhalb des verschlossenen Trichterbodens wurde ein etikettierter Packkarton 70 zur Verfügung gestellt, wobei das Etikett des Packkartons 70 eine Karton-Identifikationsnummer aufwies. Die Karton-Identifikationsnummer wurde mit einem Barcodeleser eingescannt und ausgelesen. Zuerst fiel der erste rezeptpflichtige Apothekenartikel auf dem Förderband in den Trichter. Während der erste rezeptpflichtige Apothekenartikel in den Trichter 60 viel wurden durch 12 Kameras, die wie in Figur 2 (A) bis (C) dargestellt an einem Gestell angebracht waren Fotos aufgenommen. Es wurden Bilder mit einer Frequenz von 55 Hz aufgenommen. Aus den Fotos konnte der Data Matrix Code des Etiketts des ersten rezeptpflichtigen Apothekenartikels ausgelesen werden und damit der Etikettcode, der in diesem verschlüsselt war. Weiterhin war als Identifikationscode der SecurPharm Code auf die Umverpackung des ersten rezeptpflichtigen Apothekenartikels gedruckt. Auch dieser konnte aus den Fotos entnommen werden. Der Etikettcode aus dem Data Matrix Code, der SecurPharm Code der Umverpackung und die Karton-Identifikationsnummer wurden mit den digitalen Daten verglichen, die mit dem digitalen Kommissionierauftrag verknüpft waren.

Nacheinander wurden auch der zweite und der dritte rezeptpflichtige Apothekenartikel vom Förderband 120 in den Trichter 60 abgegeben, wobei auch von diesen Fotos aufgenommen wurden. Auch für den zweiten und den dritten rezeptpflichtigen Apothekenartikel konnte jeweils der Data Matrix Code des Etiketts und der SecurPharm Code auf der Umverpackung der rezeptpflichtigen Apothekenartikel erkannt werden. Die Produktcodes aus den Data Matrix Codes, die SecurPharm Codes der Umverpackung und die Karton-Identifikationsnummer wurden mit den digitalen Daten verglichen, die mit dem digitalen Kommissionierauftrag verknüpft waren.

Als alle drei rezeptpflichtigen Apothekenartikel in dem geschlossenen Trichter 60 gesammelt waren, wurde der Trichter 60 geöffnet und sämtliche rezeptpflichtigen Apothekenartikel rutschten in den darunter stehenden Packkarton 70. Da beim Vergleich des Etikettcodes aus dem Data Matrix Code, des SecurPharm Codes der Umverpackung und der Karton-Identifikationsnummer mit den digitalen Daten, die mit dem digitalen Kommissionierauftrag verknüpft waren für keinen der drei rezeptpflichtigen Apothekenartikel eine Abweichung festgestellt werden konnte, wurde der Packkarton 70 über das Förderbad 129 zu einer Waage 80 transportiert. Dort wurde das Ist-Gewicht des Packkartons gewogen. Da das Gewicht der drei kommissionierten rezeptpflichtigen Apothekenartikel bekannt war, wurde ein Soll-Gewicht berechnet. Ist-Gewicht und Soll-Gewicht wurden miteinander verglichen. Da der leichteste Artikel im Packkarton unter 10 g wog, wurden Abweichungen des Ist-Gewichtes zum Soll-Gewicht bis zu 20 % dieses Artikels als akzeptabel angesehen. Die festgestellte Abweichung lag innerhalb des festgelegten Bereiches und der Packkarton 70 wurde verschlossen und zum Versand 200 freigegeben.

### Ausführungsbeispiel 2 - Fehlerhaftes kommissionieren von rezeptpflichtigen Apothekenartikeln

In diesem Ausführungsbeispiel wurden, wie im Ausführungsbeispiel 1, drei rezeptpflichtige Apothekenartikel kommissioniert, die durch ein elektronisches Rezept verordnet wurden. Beim Vergleich des Etikettcodes des rezeptpflichtigen Apothekenartikels der als zweites in den Trichter 60 abgelegt wurde, des SecurPharm Codes, der auf der Umverpackung dieses rezeptpflichtigen Apothekenartikels aufgebracht war und der Karton-Identifikationsnummer mit den digitalen Daten, die mit dem digitalen Kommissionierauftrag verknüpft sind, wurde eine Abweichung festgestellt. Konkret stimmten der Etikettcode aus dem Data Matrix Codes mit keinem Etikettcode in den digitalen Daten überein, die mit dem digitalen Kommissionierauftrag verknüpft waren.

Die drei rezeptpflichtigen Apothekenartikel wurden in dem Trichter 60 gesammelt und in den bereitgestellten Packkarton 70 gefüllt. Der Packkarton 70 wurde anschließend aufgrund der beschriebenen Abweichung zwischen dem Etikettcode aus dem Data Matrix Codes und den digitalen Daten, die mit dem digitalen Kommissioneirauftrag verknüpft sind, einer Kontrollstation 90 zugeführt.

### Ausführungsbeispiel 3 - Kommissionieren von rezeptpflichtigen Apothekenartikeln, nicht rezeptpflichtigen Apothekenartikeln und Drogerieartikeln

Es wurden zwei digitale Rezepte über eine App für einen Kunden eingereicht. Das erste digitale Rezept wurde eingelesen und ein digitaler Kommissionierauftrag wurde erstellt. Mit dem elektronischen Rezept wurden drei rezeptpflichtige Apothekenartikel verordnet, wobei für jeden rezeptpflichtigen Apothekenartikel eine Dosieranweisung seitens des Arztes vermerkt war. Dem digitalen Kommissionierauftrag wurden sämtliche Daten des elektronischen Rezeptes zugeordnet. Insbesondere folgende Informationen:
- Name und Adresse des Kunden;
- Rezeptpflichtige Apothekenartikel, die mit dem elektronischen Rezept verordnet wurden;
- Dosieranweisungen für jeden verordneten rezeptpflichtigen Apothekenartikel.

Das zweite digitale Rezept wurde eingelesen, wobei mit diesem zwei weitere rezeptpflichtige Apothekenartikel verordnet wurden. Sämtliche elektronische Daten des zweiten elektronischen Rezeptes wurden ebenfalls dem digitalen Kommissioneirauftrag zugeordnet.

Der erste rezeptpflichtige Apothekenartikeln wurde aus einem Warenlager 30 zur Verfügung gestellt, mit dem Etikettierer 40 etikettiert, eingescannt und in einer leeren Pufferschale 51 abgelegt. Anschließend wurde der zweite rezeptpflichtige Apothekenartikeln aus dem Warenlager 30 zur Verfügung gestellt und durchlief die gleichen Verfahrensschritte wie zuvor der erste rezeptpflichtige Apothekenartikel. Der zweite rezeptpflichtige Apothekenartikel wurde anschließend in einer leeren Pufferschale 52 abgelegt. Anschließend wurde der dritte rezeptpflichtige Apothekenartikeln aus dem Warenlager 30 zur Verfügung gestellt und durchlief die gleichen Verfahrensschritte wie zuvor der erste und zweite rezeptpflichtige Apothekenartikel. Der dritte rezeptpflichtige Apothekenartikel wurde anschließend in einer leeren Pufferschale 53 abgelegt. In gleicher Weise wurde für den vierten und fünften rezeptpflichtigen Apothekenartikel verfahren. Beim Vergleich der gescannten Daten mit den Daten, die mit dem digitalen Kommissionierauftrag verknüpft waren, wurden keine Abweichungen festgestellt.

Zusätzlich wurden über einen Online-Shop zwei nichtrezeptpflichtige Apothekenartikel und 3 Drogerieartikel durch den gleichen Kunden bestellt. Die Daten dieser Bestellung, insbesondere
- die Art und Anzahl der bestellten nichtrezeptpflichtigen Apothekenartikel,
- die Art und Anzahl der bestellten Drogerieartikel,
- Kundendaten wie Name und Adresse,
- Bestellnummern, und
- Kundennummer,
wurden mit dem digitalen Kommissionierauftrag verknüpft. In einer Pufferschale 55 wurden die zwei nichtrezeptpflichtigen Apothekenartikel und die drei Drogerieartikel bereitgestellt.

Zunächst wurden die Pufferschalen 51, 52, 53 mit den rezeptpflichtigen Apothekenartikeln wie in Ausführungsbeispiel 1 beschrieben auf das Förderband 120 geleert. Anschließend wurde zeitlich beabstandet die Pufferschale 55 mit den nichtrezeptpflichtigen Apothekenartikeln und den Drogerieartikeln auf das Förderband 120 geleert. Hierbei war zwischen dem Haufen der nichtrezeptpflichtigen Apothekenartikel und der Drogerieartikel und dem letzten rezeptpflichtigen Apothekenartikel auf dem Förderband 120 ein Abstand von 30 cm.

Alle fünf rezeptpflichtigen Apothekenartikel wurden analog wie in Ausführungsbeispiel 1 beschrieben in den Trichter 60 abgegeben. Anschließend wurde der Haufen mit den nichtrezeptpflichtigen Apothekenartikeln und Drogerieartikeln in den Trichter 60 abgegeben. Dabei wurde der Haufen durch die 12 Kameras gescannt, die bereits die Fotos von den rezeptpflichtigen Apothekenartikel bei deren Abgabe vom Förderband 120 in den Trichter 60 aufgenommen haben. Aus den Fotos der 12 Kameras konnte für einen nichtrezeptpflichtigen Apothekenartikel und einen Drogerieartikel jeweils ein Identifikationscode ausgelesen werden.

Zusätzlich zu den Etikettcodes aus den Data Matrix Codes der rezeptpflichtigen Apothekenartikel, deren SecurPharm Codes auf der Umverpackung und der Karton-Identifikationsnummer wurden auch der Identifikationscode des nichtrezeptpflichtigen Apothekenartikels und der Identifikationscode des Drogerieartikels mit den digitalen Daten verglichen, die mit dem digitalen Kommissionierauftrag verknüpft waren, verglichen.

Als die fünf rezeptpflichtigen Apothekenartikel, die zwei nichtrezeptpflichtigen Apothekenartikel und die drei Drogerieartikel in dem Trichter 60 gesammelt waren, wurde der Trichter 60 geöffnet und dessen Inhalt rutschte in den darunter bereitgestellten Packkarton 70. Da beim Vergleich der Etikettcodes aus dem Data Matrix Code der rezeptpflichtigen Apothekenartikel, der SecurPharm Codes der Umverpackung der rezeptpflichtigen Apothekenartikel, der Karton-Identifikationsnummer, des Identifikationscodes des nichtrezeptpflichtigen Apothekenartikels und des Identifikationscodes des Drogerieartikels mit den digitalen Daten, die mit dem digitalen Kommissionierauftrag verknüpft waren, keinerlei Abweichung festgestellt werden konnte, wurde der Packkarton 70 über das Förderbad 129 zu einer Waage 80 transportiert.

Dort wurde das Ist-Gewicht des Packkartons gewogen. Da das Gewicht der fünf kommissionierten rezeptpflichtigen Apothekenartikel, der zwei nichtrezeptpflichtigen Apothekenartikel und der drei Drogerieartikel bekannt war, wurde ein Soll-Gewicht berechnet. Der leichteste Artikel im Packkarton wog zwischen 10 g und 20 g, womit Abweichungen des Ist-Gewichtes zum Soll-Gewicht bis zu 50 % dieses Artikels akzeptiert werden. Ist-Gewicht und Soll-Gewicht wurden miteinander verglichen und es wurde eine Abweichung des Ist-Gewichtes vom Soll-Gewicht, innerhalb des beschriebenen Bereiches festgestellt. Der Packkarton 70 wurde daher verschlossen und zum Versand 200 freigegeben.

### Ausführungsbeispiel 4 - Sortieren von rezeptpflichtigen Apothekenartikeln

Es wurde ein Packkarton mit 50 unsortierten rezeptpflichtigen Apothekenartikeln angeliefert. Der Packkarton enthielt dabei jeweils 10 Packungen eines Typs eines rezeptpflichtig Apothekenartikels. Der Packkarton wurde geöffnet und dessen Inhalt in einen Vereinzler 10 geschüttet. Der Vereinzler 10 gab je einen rezeptpflichtigen Apothekenartikel nach dem anderen auf ein Förderband ab. Auf dem Förderband lagen die einzelnen rezeptpflichtigen Apothekenartikel beabstandet zueinander mit einem Abstand von 30 cm.

Anschließend wurde jeder Apothekenartikel von 6 Seiten gescannt. Hierfür wurde bevorzugt eine Vorrichtung 20 verwendet, bei der an einem Gestell mindestens 6 Digitalkameras befestigt sind.

Beim Einscannen wurde der SecurPharm Code auf jedem der eingescannten rezeptpflichtigen Apothekenartikel erkannt, sowie die Position des SecurPharm Codes auf jedem der eingescannten rezeptpflichtigen Apothekenartikel. Diese Daten wurden für den jeweils eingescannten rezeptpflichtigen Apothekenartikel abgespeichert und es wurde ein Etikettcode für jeden eingescannten rezeptpflichtigen Apothekenartikel erstellt. Der Etikettcode wies mindestens den SecurPharm Code des jeweiligen rezeptpflichtigen Apothekenartikels auf. Der Etikettcode wurde ebenfalls gespeichert.

Die rezeptpflichtigen Apothekenartikel wurden anschließend in einem angeschlossenen Warenlager 30 eingelagert. Eine Einlagerung wurde jedoch nur unter der Voraussetzung vorgenommen, dass sich auf der Oberseite des rezeptpflichtigen Apothekenartikels nicht der SecurPharm Code des rezeptpflichtigen Apothekenartikels befand. Als Oberseite wird dabei die Seite des rezeptpflichtigen Apothekenartikels verstanden, die sich gegenüber der Seite des rezeptpflichtigen Apothekenartikels befindet, die auf der Fördertechnik liegt. Dies traf auf alle rezeptpflichtigen Apothekenartikel in dem Packkarton zu.

In einer alternativen Ausführungsform wird der rezeptpflichtige Apothekenartikel in ein Warenlager 30 eingelagert und der Etikettcode wird erst später bei der Kommissionierung erzeugt.

## Patentansprüche

1. Verfahren zur vollautomatischen Kommissionierung von rezeptpflichtigen Apothekenartikeln aus einem Warenlager, wobei
• für jeden zu kommissionierenden rezeptpflichtigen Apothekenartikel ein Etikettcode erzeugt wird;
• ein SecurPharm Code auf jedem zu kommissionierenden rezeptpflichtigen Apothekenartikel aufgebracht ist und die Position des SecurPharm auf dem rezeptpflichtigen Apothekenartikel bekannt ist; und
aufweisend die Schritte
a) Bereitstellen eines digitalen Rezeptes über eine digitale Plattform oder eine App oder eines analogen Rezeptes und Erstellen eines digitalen Kommissionierauftrages für das digitale oder analoge Rezept mit einer Recheneinheit;
b) Zuordnen der digitalen Daten des elektronischen Rezeptes, in Form der bestellten rezeptpflichtigen Apothekenartikel, der personenbezogenen Daten auf dem elektronischen Rezept, Dosierungsanweisungen, weiterer Hinweise des Arztes, die auf dem elektronischen Rezept vermerkt sind zu dem digitalen Kommissionierauftrag mit einer Recheneinheit oder
Digitalisieren des analogen Rezeptes und Zuordnen der digitalen Daten des digitalisierten analogen Rezeptes in Form der bestellten rezeptpflichtigen Apothekenartikel, der personenbezogenen Daten auf dem elektronischen Rezept, Dosierungsanweisungen, weiterer Hinweise des Arztes, die auf dem elektronischen Rezept vermerkt sind zu dem digitalen Kommissionierauftrag mit einer Recheneinheit
c) Optional Bereitstellen mindestens eines weiteren digitalen oder analogen Rezeptes und Zuordnen der digitalen Daten des elektronischen Rezeptes, in Form der bestellten rezeptpflichtigen Apothekenartikel, der personenbezogenen Daten auf dem elektronischen Rezept, Dosierungsanweisungen, weiterer Hinweise des Arztes, die auf dem elektronischen Rezept vermerkt sind zu dem in Schritt a) erzeugten digitalen Kommissionierauftrag mit einer Recheneinheit, oder
Digitalisieren des analogen Rezeptes und Zuordnen der digitalen Daten des digitalisierten analogen Rezeptes in Form der bestellten rezeptpflichtigen Apothekenartikel, der personenbezogenen Daten auf dem elektronischen Rezept, Dosierungsanweisungen, weiterer Hinweise des Arztes, die auf dem elektronischen Rezept vermerkt sind zu dem in Schritt a) erzeugten digitalen Kommissionierauftrag mit einer Recheneinheit
d) Bereitstellen eines rezeptpflichtigen Apothekenartikels, des digitalen Kommissionierauftrages aus einem Warenlager durch einen Roboter und/oder eine Fördereinrichtung;
e) Zuordnen des Etikettcodes des bereitgestellten rezeptpflichtigen Apothekenartikels zu dem digitalen Kommissionierauftrag durch eine Recheneinheit;
f) Drucken eines Etiketts, aufweisend personenbezogene Daten des Rezeptempfängers, Angaben zur Dosierung des bereitgestellten rezeptpflichtigen Apothekenartikels, einen DataMatrix-Code aufweisend den Etikettcode des bereitgestellten rezeptpflichtigen Apothekenartikels und optional weiterer Hinweise des Arztes, die auf einem elektronischen Rezept bezüglich des rezeptpflichtigen Apothekenartikels vermerkt sind durch einen Etikettierer;
g) Applizieren des Etiketts auf einer Seite des rezeptpflichtigen Apothekenartikels derart, dass das Etikett den SecurPharm Code des rezeptpflichtigen Apothekenartikels nicht verdeckt durch den Etikettierer;
h) Einscannen des Data Matrix Codes des Etiketts und auslesen des darin enthaltenen Etikettcodes und Vergleich des Etikettcodes mit den dem digitalen Kommissionierauftrag zugeordneten Etikettcodes durch einen Scanner;
i) Ablegen des etikettierten rezeptpflichtigen Apothekenartikels in einer leeren Pufferschale, wenn im Verfahrensschritt h) festgestellt wurde, dass der Etikettcode mit einem Etikettcode, der im digitalen Kommissionierauftrag hinterlegt ist, übereinstimmt oder Ablegen des etikettierten rezeptpflichtigen Apothekenartikels in einer Fehlerschale, wenn im Verfahrensschritt h) festgestellt wurde, dass der Etikettcode nicht mit einem Etikettcode, der im digitalen Kommissionierauftrag hinterlegt ist, übereinstimmt;
j) Wiederholen der Schritte d) bis i) für alle rezeptpflichtigen Apothekenartikel, die dem digitalen Kommissionierauftrag zugeordnet sind;
k) Nacheinander Ausgabe der rezeptpflichtigen Apothekenartikel aus den Pufferschalen auf eine Fördertechnik, so dass jeder rezeptpflichtige Apothekenartikel auf der Fördertechnik mindestens einen vorgegebenen Abstand zu einem weiteren rezeptpflichtigen Apothekenartikel oder einem anderen Artikel aufweist und Transport der rezeptpflichtigen Apothekenartikel durch die Fördertechnik zu einer Packstation;
l) Bereitstellen eines etikettierten Packkartons, wobei das Etikett des Packkartons eine Karton-Identifikationsnummer aufweist und Zuordnen der Karton-Identifikationsnummer zu dem digitalen Kommissionierauftrag;
m) Scannen der Karton-Identifikationsnummer vom Etikett des Packkartons durch einen Scanner;
n) Abgabe eines auf der Fördertechnik transportierten rezeptpflichtigen Apothekenartikels in einen Trichter mit einem Kamerasystem, wobei der rezeptpflichtige Apothekenartikel von 6 Seiten gescannt wird, wobei der Data Matrix Code des Etiketts des rezeptpflichtigen Apothekenartikels erkannt wird und wobei der SecurPharm Code des rezeptpflichtigen Apothekenartikels auf dem rezeptpflichtigen Apothekenartikel erkannt wird;
o) Vergleich des im gescannten Data Matrix Codes enthaltenen Etikettcodes und des gescannten SecurPharm Codes auf dem rezeptpflichtigen Apothekenartikel und der in Verfahrensschritt m) gescannten Karton-Identifikationsnummer mit den digitalen Daten, die dem digitalen Kommissionierauftrag zugeordnet sind durch eine Recheneinheit;
p) Wiederholung der Verfahrensschritte n) bis o) für alle in Verfahrensschritt k) auf die Fördertechnik abgegebenen rezeptpflichtigen Apothekenartikel, wobei alle rezeptpflichtigen Apothekenartikel in den gleichen Trichter abgegeben werden;
q) Abgabe der im Trichter gesammelten rezeptpflichtigen Apothekenartikel in den bereitgestellten Packkarton durch Öffnen des Trichters;
r) Freigabe des Packkartons zur Versendung oder weiteren Verarbeitung und Abmeldung der SecurPharm Codes aller kommissionierten rezeptpflichtigen Apothekenartikel beim EU-Hub Netzwerk, wenn im Verfahrensschritt o) für alle zu kommissionierenden rezeptpflichtigen Apothekenartikel festgestellt wurde, dass der Etikettcode und der SecurPharm Codes mit einem Etikettcode und einem SecurPharm Code übereinstimmen, die im digitalen Kommissionierauftrag hinterlegt sind und die gescannte Karton-Identifikationsnummer mit der Karton-Identifikationsnummer übereinstimmt, die im digitalen Kommissionierauftrag hinterlegt ist;
oder Transport des Packkartons zu einer Fehlerkontrolle, wenn im Verfahrensschritt o) für zumindest einen zu kommissionierenden rezeptpflichtigen Apothekenartikel festgestellt wurde, dass der Etikettcode oder der SecurPharm Code nicht mit einem Etikettcode oder einem SecurPharm Code übereinstimmen, die im digitalen Kommissionierauftrag hinterlegt sind;
oder Transport des Packkartons zu einer Fehlerkontrolle, wenn die gescannte Karton-Identifikationsnummer nicht mit der Karton-Identifikationsnummer übereinstimmt, die im digitalen Kommissionierauftrag hinterlegt ist.

2. Verfahren gemäß Anspruch 1, **dadurch gekennzeichnet, dass** das Verfahren weiterhin das kommissionieren von nichtrezeptpflichtigen Apothekenartikeln und/oder Drogerieartikeln aus einem Warenlager umfasst, wobei für jeden nichtrezeptpflichtigen Apothekenartikel und/oder Drogerieartikel ein oder mehrere Identifikationscodes bekannt sind, durch die der zu kommissionierenden nichtrezeptpflichtige Apothekenartikel und/oder Drogerieartikel eindeutig identifiziert werden kann, und wobei mindestens ein Identifikationscode auf jedem zu kommissionierenden nichtrezeptpflichtigen Apothekenartikel und/oder Drogerieartikel oder auf dessen Umverpackung aufgebracht ist, wobei das Verfahren weiterhin die Schritte aufweist
aa) Bereitstellen eines digitalen oder analogen Rezeptes und Erstellen eines digitalen Kommissionierauftrages für das digitale oder analoge Rezept;
ab) Einlesen des digitalen oder analogen Rezeptes und erkennen der durch das Rezept bestellten rezeptpflichtigen Apothekenartikel, der personenbezogenen Daten auf dem elektronischen Rezept, Dosierungsanweisungen, weiterer Hinweise des Arztes, die auf dem elektronischen Rezept vermerkt sind und erkennen der durch das Rezept bestellten nichtrezeptpflichtigen Apothekenartikel und/oder Drogerieartikel;
ac) Zuordnen der in Schritt ab) erkannten digitalen Daten zu dem digitalen Kommissionierauftrag und optional zuordnen weiterer Bestellungen von nichtrezeptpflichtigen Apothekenartikel und/oder Drogerieartikel;
ad) Optional bereitstellen mindestens eines weiteren digitalen oder analogen Rezeptes und einlesen des mindestens einen weiteren digitalen oder analogen Rezeptes und erkennen der durch das mindestens eine weitere digitale oder analoge Rezept bestellten rezeptpflichtigen Apothekenartikel, der personenbezogenen Daten auf dem mindestens einen weiteren digitalen oder analogen Rezept, Dosierungsanweisungen, weiterer Hinweise des Arztes, die auf dem mindestens einen weiteren digitalen oder analogen Rezept vermerkt sind und erkennen der durch das mindestens eine weitere digitalen oder analogen Rezept bestellten nichtrezeptpflichtigen Apothekenartikel und/oder Drogerieartikel; und Zuordnen der erkannten digitalen Daten zu dem in Schritt aa) erzeugten digitalen Kommissionierauftrag;
ae) Bereitstellen eines rezeptpflichtigen Apothekenartikels, des digitalen Kommissionierauftrages aus einem Warenlager durch einen Roboter und/oder eine Fördereinrichtung;
af) Zuordnen des Etikettcodes des bereitgestellten rezeptpflichtigen Apothekenartikels zu dem digitalen Kommissionierauftrag;
ag) Drucken eines Etiketts, aufweisend personenbezogene Daten des Rezeptempfängers, Angaben zur Dosierung des bereitgestellten rezeptpflichtigen Apothekenartikels, eines DataMatrix-Codes aufweisend den Etikettcode des bereitgestellten rezeptpflichtigen Apothekenartikels und optional weiterer Hinweise des Arztes, die auf einem elektronischen Rezept bezüglich des rezeptpflichtigen Apothekenartikels vermerkt sind;
ah) Applizieren des Etiketts auf einer Seite des rezeptpflichtigen Apothekenartikels derart, dass das Etikett den SecurPharm Code des rezeptpflichtigen Apothekenartikels nicht verdeckt;
ai) Einscannen des Data Matrix Codes des Etiketts und auslesen des darin enthaltenen Etikettcodes und Vergleich des Etikettcodes mit den dem digitalen Kommissionierauftrag zugeordneten Etikettcodes;
aj) Ablegen des etikettierten rezeptpflichtigen Apothekenartikels in einer leeren Pufferschale, wenn im Verfahrensschritt ai) festgestellt wurde, dass der Etikettcode mit einem Etikettcode, der im digitalen Kommissionierauftrag hinterlegt ist, übereinstimmt oder Ablegen des etikettierten rezeptpflichtigen Apothekenartikels in einer Fehlerschale, wenn im Verfahrensschritt ai) festgestellt wurde, dass der Etikettcode nicht mit einem Etikettcode, der im digitalen Kommissionierauftrag hinterlegt ist, übereinstimmt;
ak) Wiederholen der Schritte ae) bis aj) für alle rezeptpflichtigen Apothekenartikel, die dem digitalen Kommissionierauftrag zugeordnet sind;
al) Bereitstellen aller im digitalen Kommissionierauftrag enthaltenen nichtrezeptpflichtigen Apothekenartikel und/oder Drogerieartikel in einer Pufferschale;
am) Nacheinander Ausgabe der rezeptpflichtigen Apothekenartikel aus den Pufferschalen auf eine Fördertechnik, so dass jeder rezeptpflichtige Apothekenartikel auf der Fördertechnik mindestens einen vorgegebenen Abstand zu einem weiteren rezeptpflichtigen Apothekenartikel oder einem anderen Artikel aufweist und Transport der rezeptpflichtigen Apothekenartikel durch die Fördertechnik zu einer Packstation;
an) Abgabe der nichtrezeptpflichtigen Apothekenartikel und/oder Drogerieartikel als Haufen aus der Pufferschale auf die Fördertechnik aus Verfahrensschritt am);
ao) Bereitstellen eines etikettierten Packkartons, wobei das Etikett des Packkartons eine Karton-Identifikationsnummer aufweist und Zuordnen der Karton-Identifikationsnummer zu dem digitalen Kommissionierauftrag;
ap) Scannen der Karton-Identifikationsnummer vom Etikett des Packkartons;
aq) Abgabe eines auf der Fördertechnik transportierten rezeptpflichtigen Apothekenartikels in einen Trichter, wobei der rezeptpflichtige Apothekenartikel von 6 Seiten gescannt wird, wobei der Data Matrix Code des Etiketts des rezeptpflichtigen Apothekenartikels erkannt wird und wobei der SecurPharm Code des rezeptpflichtigen Apothekenartikels auf dem rezeptpflichtigen Apothekenartikel erkannt wird;
ar) Vergleich des im gescannten Data Matrix Codes enthaltenen Etikettcodes und des gescannten SecurPharm Codes auf dem rezeptpflichtigen Apothekenartikel und der in Verfahrensschritt ap) gescannten Karton-Identifikationsnummer mit den digitalen Daten, die dem digitalen Kommissionierauftrag zugeordnet sind;
as) Wiederholung der Verfahrensschritte aq) bis ar) für alle in Verfahrensschritt am) auf die Fördertechnik abgegebenen rezeptpflichtigen Apothekenartikel, wobei alle rezeptpflichtigen Apothekenartikel in den gleichen Trichter abgegeben werden;
at) Abgabe des Haufens der nichtrezeptpflichtigen Apothekenartikel und/oder Drogerieartikel von der Fördertechnik in den Trichter, wobei der Haufen der nichtrezeptpflichtigen Apothekenartikel und/oder Drogerieartikel von 6 Seiten gescannt wird, wobei ein oder mehrere Identifikationscodes von Herstellern von nichtrezeptpflichtigen Apothekenartikel und/oder Drogerieartikel erkannt werden können;
au) Vergleich jedes Identifikationscodes eines Herstellers von einem nichtrezeptpflichtigen Apothekenartikel und/oder Drogerieartikel, der im Verfahrensschritt at) erkannt wird mit den Identifikationscodes der Hersteller, die im digitalen Kommissionierauftrag hinterlegt sind;
av) Abgabe der im Trichter gesammelten rezeptpflichtigen Apothekenartikel und nichtrezeptpflichtigen Apothekenartikel und/oder Drogerieartikel in den bereitgestellten Packkarton;
aw)Verschließen und Freigabe des Packkartons zur Versendung und Abmeldung der SecurPharm Codes der kommissionierten rezeptpflichtigen Apothekenartikel beim EU-Hub Netzwerk, wenn im Verfahrensschritt ar) für alle zu kommissionierenden rezeptpflichtigen Apothekenartikel festgestellt wurde, dass der Etikettcode und der SecurPharm Codes mit einem Etikettcode und einem SecurPharm Code übereinstimmen, die im digitalen Kommissionierauftrag hinterlegt sind und die gescannte Karton-Identifikationsnummer mit der Karton-Identifikationsnummer übereinstimmt, die im digitalen Kommissionierauftrages hinterlegt ist und wenn alle im Verfahrensschritt au) verglichenen Identifikationscodes von Herstellern im digitalen Kommissionierauftrag hinterlegt sind;
oder Transport des Packkartons zu einer Fehlerkontrolle, wenn im Verfahrensschritt ar) für zumindest einen zu kommissionierenden rezeptpflichtigen Apothekenartikel festgestellt wurde, dass der Etikettcode oder der SecurPharm Code nicht mit einem Etikettcode oder einem SecurPharm Code übereinstimmen, die im digitalen Kommissionierauftrag hinterlegt sind;
oder Transport des Packkartons zu einer Fehlerkontrolle, wenn ein im Verfahrensschritt au) verglichener Identifikationscode eines Herstellers nicht im digitalen Kommissionierauftrag hinterlegt ist;
oder Transport des Packkartons zu einer Fehlerkontrolle, wenn die gescannte Karton-Identifikationsnummer nicht mit der Karton-Identifikationsnummer übereinstimmt, die im digitalen Kommissionierauftrag hinterlegt ist.

3. Verfahren gemäß einem der vorangegangenen Ansprüche, **dadurch gekennzeichnet, dass** weiterhin das Gewicht jedes zu kommissionierenden rezeptpflichtigen Apothekenartikels und nichtrezeptpflichtigen Apothekenartikels und Drogerieartikels bekannt ist und dass die für die im Verfahrensschritt r) oder aw) für eine Kontrolle ausgegebenen Packkartons folgende Verfahrensschritte durchlaufen
I. Transport des Packkartons zu einer Kontrollstation;
II. Scannen der Karton-Identifikationsnummer und Vergleich dieser mit den mit dem digitalen Kommissionierauftrag verknüpften digitalen Daten;
III. Auskippen des Inhalts des Packkartons in einen Behälter;
IV. Aufnahme eines rezeptpflichtigen Apothekenartikels oder eines nichtrezeptpflichtigen Apothekenartikels oder eines Drogerieartikels aus dem Behälter durch einen Roboter und Einscannen des Apothekenartikels von mindestens 6 Seiten, wobei bei rezeptpflichtigen Apothekenartikeln der Data Matrix Code des Etikettes und der SecurPharm Code auf dem rezeptpflichtigen Apothekenartikel gescannt wird und bei nichtrezeptpflichtigen Apothekenartikeln oder Drogerieartikeln der mindestens eine Identifikationscode des Herstellers auf dem nichtrezeptpflichtigen Apothekenartikel oder Drogerieartikel gescannt wird;
V. Vergleich des Etikettcodes und des SecurPharm Codes auf dem rezeptpflichtigen Apothekenartikel mit den digitalen Daten, die dem digitalen Kommissionierauftrag zugeordnet sind; oder Vergleich des mindestens einen gescannten Identifikationscodes des Herstellers mit den digitalen Daten, die dem digitalen Kommissionierauftrag zugeordnet sind für einen nichtrezeptpflichtigen Apothekenartikel oder einen Drogerieartikel;
VI. Ablegen des etikettierten rezeptpflichtigen Apothekenartikels oder des rezeptfreien Apothekenartikels oder des Drogerieartikels in dem Packkarton, wenn in Schritt V. und in Schritt II. die gescannten Daten mit den digitalen Daten, die dem digitalen Kommissionierauftrag zugeordnet sind, übereinstimmen; oder Auswurf in eine Fehlerschale wenn in Schritt V. oder in Schritt II. die gescannten Daten nicht mit den digitalen Daten des digitalen Kommissionierauftrages übereinstimmen;
VII. Wiederholen der Schritte IV. bis VI. für alle in Verfahrensschritt III. ausgekippten rezeptpflichtigen Apothekenartikel, nichtrezeptpflichtigen Apothekenartikel und Drogerieartikel;
VIII. Transport des Packkartons auf einer Fördertechnik zu einer Waage und wiegen des Packkartons, wenn im Verfahrensschritt VI. kein Apothekenartikel und/oder Drogerieartikel in eine Fehlerschale ausgeworfen wurde, wobei das ermittelte Gewicht das Ist-Gewicht des Packkartons ist;
IX. Berechnen des Soll-Gewichtes des Packkartons und Vergleich des Ist-Gewichts des Packkartons mit dem Soll-Gewicht des Packkartons;
X. Verschließen und Freigabe des Packkartons zur Versendung, wenn in Schritt IX. eine Abweichung von Soll- und Ist-Gewicht innerhalb eines vorgegebenen Toleranzbereichs festgestellt wird oder Ausgabe des Packkartons auf eine Fördertechnik für eine weitere Kontrolle, wenn in Schritt IX. eine Abweichung von Soll- und Ist-Gewicht außerhalb eines vorgegebenen Toleranzbereichs festgestellt wird.

4. Verfahren nach einem der vorangegangenen Ansprüche, **dadurch gekennzeichnet, dass** die rezeptpflichtigen Apothekenartikel bevorzugt quaderförmig sind.

5. Verfahren nach Anspruch 3, **dadurch gekennzeichnet, dass** zwischen 0% und 100% der kommissionierten Packkartons, bevorzugt zwischen 0% und 25% der kommissionierten Packkartons, besonders bevorzugt zwischen 0% und 5 % der kommissionierten Packkartons nach dem Verfahrensschritt r) oder aw) auf die Fördertechnik für eine zusätzliche Kontrolle ausgegeben werden, wobei die Ausgegebenen Packkartons für den Versand oder die Weiterbearbeitung vorgesehen waren.

6. Verfahren nach einem der vorangegangenen Ansprüche, **dadurch gekennzeichnet, dass** in Verfahrensschritt n), at) oder aq) weiterhin ein Foto oder mehrere Fotos von den zu kommissionierenden Apothekenartikeln und/oder dem Inhalt des Packkartons aufgenommen werden.

7. Verfahren nach einem der vorangegangenen Ansprüche, **dadurch gekennzeichnet, dass** ein Foto oder mehrere Fotos von dem Inhalt eines vollständige kommissionierten Packkartons aufgenommen werden, bevor dieser für den Versand verschlossen wird.

8. Verfahren nach einem der vorangegangenen Ansprüche, **dadurch gekennzeichnet, dass** der securPharm Code jedes der in einen Packkarton kommissionierten apothekenpflichtigen Apothekenartikel bei Versand des Kartons automatisch in der securPharm Datenbank abgemeldet wird, wobei ein Zeitstempel für die Abmeldung in der securPharm Datenbank erzeugt wird.

9. Verfahren nach Anspruch 8, **dadurch gekennzeichnet, dass** die Daten des Etiketts und der Zeitstempel für die Abmeldung in der securPharm Datenbank untereinander und mit dem digitalen Kommissionierauftrag verknüpft und gespeichert werden.

10. Verfahren gemäß einem der vorangegangenen Ansprüche, **dadurch gekennzeichnet, dass** das Verfahren weiterhin das Sortieren von rezeptpflichtigen Apothekenartikeln umfasst.

11. Verfahren gemäß Anspruch 10, **dadurch gekennzeichnet, dass** das Verfahren weiterhin die Schritte aufweist
• Bereitstellen einer Vielzahl von rezeptpflichtigen Apothekenartikeln;
• Vereinzeln der Vielzahl von rezeptpflichtigen Apothekenartikeln auf einer Fördertechnik, so dass jeder der rezeptpflichtigen Apothekenartikel mindestens einen vorgegebenen Abstand zu einem weiteren rezeptpflichtigen Apothekenartikel aufweist;
• Einscannen jedes der bereitgestellten rezeptpflichtigen Apothekenartikel von 6 Seiten;
• Erkennung des SecurPharm Codes auf jedem der eingescannten rezeptpflichtigen Apothekenartikel und von dessen Position auf jedem der eingescannten rezeptpflichtigen Apothekenartikel;
• Abspeichern des einen oder mehrerer Identifikationscodes eines Herstellers sowie der Position des SecurPharm Codes auf einem rezeptpflichtigen Apothekenartikel für den jeweils eingescannten rezeptpflichtigen Apothekenartikel und optional Erstellen eines Etikettcodes für jeden eingescannten rezeptpflichtigen Apothekenartikel;
• Einlagern der rezeptpflichtigen Apothekenartikel in einem Warenlager, bei denen sich der SecurPharm Code nicht auf der Oberseite des rezeptpflichtigen Apothekenartikels befindet;
• Ausgabe des rezeptpflichtigen Apothekenartikels in eine Fehlerschale, bei denen sich der SecurPharm Code auf der Oberseite des rezeptpflichtigen Apothekenartikels befindet.

12. Vorrichtung zur Durchführung des Verfahrens gemäß einem der Ansprüche 1 bis 11 aufweisend
• Mindestens eine Recheneinheit;
• Mindestens eine Etikettier Maschine;
• Mindestens eine Fördertechnik mit Anschluss an ein Warenlager;
• Einen Trichter mit einem Kamerasystem, wobei das Kamerasystem mindestens 6 Kameras aufweist;
• Mindestens eine zweite Fördertechnik;
• Mindestens ein Scanner;
• Optional mindestens eine Waage;
• Optional mindestens einen Roboter mit einer Greifeinheit.

13. Vorrichtung nach Anspruch 12, **dadurch gekennzeichnet, dass** die Vorrichtung weiterhin umfasst
• Mindestens einen Vereinzler;
• Mindestens eine weitere Fördertechnik;
• Mindestens eine Station an der ein rezeptpflichtiger Apothekenartikel von 6 Seiten gescannt werden kann;
• Ein Warenlager.

## Claims

1. Method for fully automatically picking prescription-only pharmacy products from a warehouse, wherein
• a label code is generated for each prescription-only pharmacy product to be picked;
• a SecurPharm code is applied to each prescription-only pharmacy product to be picked and the position of the SecurPharm on the prescription-only pharmacy product is known; and
comprising the steps of
a) providing a digital prescription via a digital platform or an app or an analogue prescription and creating a digital picking order for the digital or analogue prescription using a computing unit;
b) assigning the digital data of the electronic prescription, in the form of the ordered prescription-only pharmacy products, the personal data on the electronic prescription, dosage instructions, and further instructions from the doctor, which are noted on the electronic prescription, to the digital picking order using a computing unit or
digitizing the analogue prescription and assigning the digital data of the digitized analogue prescription in the form of the ordered prescription-only pharmacy products, the personal data on the electronic prescription, dosage instructions, and further instructions from the doctor, which are noted on the electronic prescription, to the digital picking order using a computing unit
c) optionally providing at least one further digital or analogue prescription and assigning the digital data of the electronic prescription, in the form of the ordered prescription-only pharmacy products, the personal data on the electronic prescription, dosage instructions, and further instructions from the doctor, which are noted on the electronic prescription, to the digital picking order generated in step a) using a computing unit, or
digitizing the analogue prescription and assigning the digital data of the digitized analogue prescription in the form of the ordered prescription-only pharmacy products, the personal data on the electronic prescription, dosage instructions, and further instructions from the doctor, which are noted on the electronic prescription, to the digital picking order generated in step a) using a computing unit
d) providing a prescription-only pharmacy product, the digital picking order from a warehouse using a robot and/or a conveyor system;
e) assigning the label code of the provided prescription-only pharmacy product to the digital picking order using a computing unit;
f) printing a label containing personal data of the prescription recipient, information on the dosage of the provided prescription-only pharmacy product, a Data Matrix code containing the label code of the provided prescription-only pharmacy product and, optionally, further instructions from the doctor, which are noted on an electronic prescription, regarding the prescription-only pharmacy product using a labeler;
g) applying the label to one side of the prescription-only pharmacy product in such a way that the label does not obscure the SecurPharm code of the prescription-only pharmacy product using the labeler;
h) scanning in the Data Matrix code of the label and reading the label code contained therein and comparing the label code with the label codes assigned to the digital picking order using a scanner;
i) placing the labeled prescription-only pharmacy product in an empty buffer tray if it was determined in method step h) that the label code matches a label code stored in the digital picking order or placing the labeled prescription-only pharmacy product in an error tray if it was determined in method step h) that the label code does not match a label code stored in the digital picking order;
j) repeating steps d) to i) for all prescription-only pharmacy products assigned to the digital picking order;
k) sequentially dispensing the prescription-only pharmacy products from the buffer trays onto a conveyor system so that each prescription-only pharmacy product on the conveyor system is at least a specified distance from another prescription-only pharmacy product or another product and transporting the prescription-only pharmacy products by means of the conveyor system to a packing station;
l) providing a labeled packing carton, wherein the label of the packing carton has a carton identification number and assigning the carton identification number to the digital picking order;
m) scanning the carton identification number from the packing carton label using a scanner;
n) dispensing a prescription-only pharmacy product transported on the conveyor system into a hopper with a camera system, wherein the prescription-only pharmacy product is scanned from 6 sides, wherein the Data Matrix code of the label of the prescription-only pharmacy product is recognized and wherein the SecurPharm code of the prescription-only pharmacy product is recognized on the prescription-only pharmacy product;
o) comparing the label code contained in the scanned Data Matrix code and the scanned SecurPharm code on the prescription-only pharmacy product and the carton identification number scanned in method step m) with the digital data assigned to the digital picking order by a computing unit;
p) repeating method steps n) to o) for all prescription-only pharmacy products dispensed onto the conveyor system in method step k), wherein all prescription-only pharmacy products are dispensed into the same hopper;
q) dispensing the prescription-only pharmacy products collected in the hopper into the provided packing carton by opening the hopper;
r) releasing the packing carton for dispatch or further processing and deregistering the SecurPharm codes of all picked prescription-only pharmacy products from the EU Hub Network if it was determined in method step o) for all prescription-only pharmacy products to be picked that the label code and the SecurPharm code match a label code and a SecurPharm code that are stored in the digital picking order and the scanned carton identification number matches the carton identification number that is stored in the digital picking order;
or transporting the packing carton for error checking if it was determined in method step o) for at least one prescription-only pharmacy product to be picked that the label code or the SecurPharm code does not match a label code or a SecurPharm code stored in the digital picking order;
or transporting the packing carton for error checking if the scanned carton identification number does not match the carton identification number stored in the digital picking order.

2. Method according to claim 1, **characterized in that** the method further comprises picking non-prescription pharmacy products and/or drugstore products from a warehouse, wherein for each non-prescription pharmacy product and/or drugstore product, one or more identification codes are known, by which the non-prescription pharmacy product and/or drugstore product to be picked can be uniquely identified, and wherein at least one identification code is applied to each non-prescription pharmacy product and/or drugstore product to be picked or to the outer packaging thereof, wherein the method further comprises the steps of
aa) providing a digital or analogue prescription and creating a digital picking order for the digital or analogue prescription;
ab) reading in the digital or analogue prescription and recognizing the prescription-only pharmacy products ordered by the prescription, the personal data on the electronic prescription, dosage instructions, and further instructions from the doctor, which are noted on the electronic prescription, and recognizing the non-prescription pharmacy products and/or drugstore products ordered by the prescription;
ac) assigning the digital data recognized in step ab) to the digital picking order and, optionally, assigning further orders for non-prescription pharmacy products and/or drugstore products;
ad) optionally providing at least one further digital or analogue prescription and reading in the at least one further digital or analogue prescription and recognizing the prescription-only pharmacy products ordered by the at least one further digital or analogue prescription, the personal data on the at least one further digital or analogue prescription, dosage instructions, and further instructions from the doctor, which are noted on the at least one further digital or analogue prescription, and recognizing the non-prescription pharmacy products and/or drugstore products ordered by the at least one further digital or analogue prescription; and assigning the recognized digital data to the digital picking order generated in step aa);
ae) providing a prescription-only pharmacy product, the digital picking order from a warehouse by a robot and/or a conveyor system;
af) assigning the label code of the provided prescription-only pharmacy product to the digital picking order;
ag) printing a label containing personal data of the prescription recipient, information on the dosage of the provided prescription-only pharmacy product, a Data Matrix code containing the label code of the provided prescription-only pharmacy product and, optionally, further instructions from the doctor, which are noted on an electronic prescription, relating to the prescription-only pharmacy product;
ah) applying the label to one side of the prescription-only pharmacy product in such a way that the label does not obscure the SecurPharm code of the prescription-only pharmacy product;
ai) scanning in the Data Matrix code of the label and reading the label code contained therein and comparing the label code with the label codes assigned to the digital picking order;
aj) placing the labeled prescription-only pharmacy product in an empty buffer tray if it was determined in method step ai) that the label code matches a label code stored in the digital picking order or placing the labeled prescription-only pharmacy product in an error tray if it was determined in method step ai) that the label code does not match a label code stored in the digital picking order;
ak) repeating steps ae) to aj) for all prescription-only pharmacy products assigned to the digital picking order;
al) providing all non-prescription pharmacy products and/or drugstore products contained in the digital picking order in a buffer tray;
am) sequentially dispensing the prescription-only pharmacy products from the buffer trays onto a conveyor system so that each prescription-only pharmacy product on the conveyor system is at least a specified distance from another prescription-only pharmacy product or another product and transporting the prescription-only pharmacy products by means of the conveyor system to a packing station;
an) dispensing the non-prescription pharmacy products and/or drugstore products as a pile from the buffer tray onto the conveyor system from method step am);
ao) providing a labeled packing carton, wherein the label of the packing carton has a carton identification number and assigning the carton identification number to the digital picking order;
ap) scanning the carton identification number from the packing carton label;
aq) dispensing a prescription-only pharmacy product transported on the conveyor system into a hopper, wherein the prescription-only pharmacy product is scanned from 6 sides, wherein the Data Matrix code of the label of the prescription-only pharmacy product is recognized and wherein the SecurPharm code of the prescription-only pharmacy product on the prescription-only pharmacy product is recognized;
ar) comparing the label code contained in the scanned Data Matrix code and the scanned SecurPharm code on the prescription-only pharmacy product and the carton identification number scanned in method step ap) with the digital data assigned to the digital picking order;
as) repeating method steps aq) to ar) for all prescription-only pharmacy products dispensed onto the conveyor system in method step am), wherein all prescription-only pharmacy products are dispensed into the same hopper;
at) discharging the pile of non-prescription pharmacy products and/or drugstore products from the conveyor system into the hopper, wherein the pile of non-prescription pharmacy products and/or drugstore products is scanned from 6 sides, wherein one or more identification codes of manufacturers of non-prescription pharmacy products and/or drugstore products can be recognized;
au) comparing each manufacturer identification code of a non-prescription pharmacy product and/or drugstore product, which is recognized in method step at), with the manufacturer identification codes stored in the digital picking order;
av) dispensing the prescription-only pharmacy products and non-prescription pharmacy products and/or drugstore products collected in the hopper into the provided packing carton;
aw) sealing and releasing the packing carton for dispatch and deregistering the SecurPharm codes of the picked prescription-only pharmacy products from the EU Hub Network if it was determined in method step ar) for all prescription-only pharmacy products to be picked that the label code and the SecurPharm code match a label code and a SecurPharm code that are stored in the digital picking order and the scanned carton identification number matches the carton identification number that is stored in the digital picking order and if all manufacturer identification codes compared in method step au) are stored in the digital picking order;
or transporting the packing carton for error checking if, in method step ar), it was determined for at least one prescription-only pharmacy product to be picked that the label code or the SecurPharm code does not match a label code or a SecurPharm code stored in the digital picking order;
or transporting the packing carton for error checking if a manufacturer identification code compared in method step au) is not stored in the digital picking order;
or transporting the packing carton for error checking if the scanned carton identification number does not match the carton identification number stored in the digital picking order.

3. Method according to either of the preceding claims, **characterized in that** furthermore, the weight of each prescription-only pharmacy product and non-prescription pharmacy product and drugstore product to be picked is known **and in that** the packing cartons dispensed for inspection in method step r) or aw) undergo the following method steps
I. transporting the packing carton to an inspection station;
II. scanning the carton identification number and comparing it with the digital data linked to the digital picking order;
III. emptying the contents of the packing carton into a container;
IV. picking up a prescription-only pharmacy product or a non-prescription pharmacy product or a drugstore product from the container by a robot and scanning in the pharmacy product from at least 6 sides, wherein in the case of prescription-only pharmacy products the Data Matrix code of the label and the SecurPharm code on the prescription-only pharmacy product are scanned and in the case of non-prescription pharmacy products or drugstore products the at least one manufacturer identification code on the non-prescription pharmacy product or drugstore product is scanned;
V. comparing the label code and the SecurPharm code on the prescription-only pharmacy product with the digital data associated with the digital picking order; or comparing the at least one scanned manufacturer identification code with the digital data associated with the digital picking order for a non-prescription pharmacy product or a drugstore product;
VI. placing the labeled prescription-only pharmacy product or the non-prescription pharmacy product or the drugstore product in the packing carton if, in step V and step II, the scanned data match the digital data assigned to the digital picking order; or ejecting it into an error tray if, in step V or step II, the scanned data do not match the digital data of the digital picking order;
VII. repeating steps IV to VI for all prescription-only pharmacy products, non-prescription pharmacy products and drugstore products dispensed in method step III;
VIII. transporting the packing carton on a conveyor system to a scale and weighing the packing carton if no pharmacy product and/or drugstore product was ejected into an error tray in method step VI, wherein the determined weight is the actual weight of the packing carton;
IX. calculating the target weight of the packing carton and comparing the actual weight of the packing carton with the target weight of the packing carton;
X. sealing and releasing the packing carton for dispatch if a deviation between the target and actual weight within a specified tolerance range is detected in step IX, or dispensing the packing carton onto a conveyor system for further inspection if a deviation between the target and actual weight outside a specified tolerance range is detected in step IX.

4. Method according to any of the preceding claims, **characterized in that** the prescription-only pharmacy products are preferably cuboidal.

5. Method according to claim 3, **characterized in that** between 0% and 100% of the picked packing cartons, preferably between 0% and 25% of the picked packing cartons, particularly preferably between 0% and 5% of the picked packing cartons are dispensed onto the conveyor system for additional inspection after method step r) or aw), wherein the dispensed packing cartons were intended for dispatch or further processing.

6. Method according to any of the preceding claims, **characterized in that** in method step n), at) or aq), one or more photos are taken of the pharmacy products to be picked and/or the contents of the packing carton.

7. Method according to any of the preceding claims, **characterized in that** one or more photos are taken of the contents of a fully picked packing carton before it is sealed for shipping.

8. Method according to any of the preceding claims, **characterized in that** the securPharm code of each of the prescription-only pharmacy products picked in a packing carton is automatically deregistered in the securPharm database when the carton is dispatched, wherein a time stamp for the deregistration is generated in the securPharm database.

9. Method according to claim 8, **characterized in that** the data of the label and the time stamp for the deregistration are linked and stored in the securPharm database with each other and with the digital picking order.

10. Method according to any of the preceding claims, **characterized in that** the method further comprises sorting prescription-only pharmacy products.

11. Method according to claim 10, **characterized in that** the method further comprises the steps of
• providing a plurality of prescription-only pharmacy products;
• separating the plurality of prescription-only pharmacy products on a conveyor system so that each of the prescription-only pharmacy products is at least a specified distance from another prescription-only pharmacy product;
• scanning in each of the provided prescription-only pharmacy products from 6 sides;
• recognizing the SecurPharm code on each of the scanned in prescription-only pharmacy products and the position thereof on each of the scanned in prescription-only pharmacy products;
• storing one or more manufacturer identification codes and the position of the SecurPharm code on a prescription-only pharmacy product for each scanned in prescription-only pharmacy product and, optionally, creating a label code for each scanned in prescription-only pharmacy product;
• storing prescription-only pharmacy products in a warehouse, for which products the SecurPharm code is not located on the top of the prescription-only pharmacy product;
• dispensing the prescription-only pharmacy product into an error tray, for which products the SecurPharm code is located on the top of the prescription-only pharmacy product.

12. Device for carrying out the method according to any of claims 1 to 11, comprising
• at least one computing unit;
• at least one labeling machine;
• at least one conveying system with connection to a warehouse;
• a hopper having a camera system, wherein the camera system has at least 6 cameras;
• at least one second conveyor system;
• at least one scanner;
• optionally at least one scale;
• optionally at least one robot having a gripping unit.

13. Device according to claim 12, **characterized in that** the device further comprises
• at least one separator;
• at least one additional conveying system;
• at least one station at which a prescription-only pharmacy product can be scanned from 6 sides;
• a warehouse.

## Revendications

1. Procédé pour la préparation entièrement automatique de commandes d'articles de pharmacie délivrés sur ordonnance à partir d'un entrepôt, dans lequel
• un code d'étiquette est généré pour chaque article de pharmacie délivré sur ordonnance à préparer pour une commande ;
• un code securPharm est apposé sur chaque article de pharmacie délivré sur ordonnance à préparer pour une commande et la position du code securPharm sur l'article de pharmacie délivré sur ordonnance est connue ; et
présentant les étapes consistant à
a) mettre à disposition une ordonnance numérique par l'intermédiaire d'une plateforme numérique ou d'une application ou une ordonnance sur papier et créer un ordre de préparation de commande numérique pour l'ordonnance numérique ou sur papier à l'aide d'une unité de calcul ;
b) associer les données numériques de l'ordonnance électronique, sous la forme des articles de pharmacie commandés délivrés sur ordonnance, des données personnelles figurant sur l'ordonnance électronique, des instructions de dosage et d'autres indications du médecin notées sur l'ordonnance électronique à l'ordre de préparation de commande numérique à l'aide d'une unité de calcul ou
numériser l'ordonnance sur papier et associer les données numériques de l'ordonnance sur papier numérisée sous la forme des articles de pharmacie commandés délivrés sur ordonnance, des données personnelles figurant sur l'ordonnance électronique, des instructions de dosage et d'autres indications du médecin notées sur l'ordonnance électronique à l'ordre de préparation de commande numérique à l'aide d'une unité de calcul
c) éventuellement, mettre à disposition au moins une autre ordonnance numérique ou sur papier et associer les données numériques de l'ordonnance électronique, sous la forme des articles de pharmacie commandés délivrés sur ordonnance, des données personnelles figurant sur l'ordonnance électronique, des instructions de dosage et d'autres indications du médecin notées sur l'ordonnance électronique à l'ordre de préparation de commande numérique généré à l'étape a) à l'aide d'une unité de calcul, ou
numériser l'ordonnance sur papier et associer les données numériques de l'ordonnance sur papier numérisée sous forme des articles de pharmacie commandés délivrés sur ordonnance, des données personnelles figurant sur l'ordonnance électronique, des instructions de dosage et d'autres indications du médecin notées sur l'ordonnance électronique à l'ordre de préparation de commande numérique généré à l'étape a) à l'aide d'une unité de calcul
d) mettre à disposition un article de pharmacie délivré sur ordonnance, l'ordre de préparation de commande numérique à partir d'un entrepôt au moyen d'un robot et/ou d'un dispositif de transport ;
e) associer, à l'aide d'une unité de calcul, le code d'étiquette de l'article de pharmacie délivré sur ordonnance et mis à disposition à l'ordre de préparation de commande numérique ;
f) imprimer, au moyen d'une étiqueteuse, une étiquette présentant des données personnelles du destinataire de l'ordonnance, des indications concernant le dosage de l'article de pharmacie délivré sur ordonnance et mis à disposition, un code DataMatrix présentant le code d'étiquette de l'article de pharmacie délivré sur ordonnance et mis à disposition et éventuellement d'autres indications du médecin notées sur une ordonnance électronique et concernant l'article de pharmacie délivré sur ordonnance ;
g) appliquer, au moyen de l'étiqueteuse, l'étiquette sur un côté de l'article de pharmacie délivré sur ordonnance de telle sorte que l'étiquette ne masque pas le code securPharm de l'article de pharmacie délivré sur ordonnance ;
h) au moyen d'un scanner, scanner le code DataMatrix de l'étiquette et lire le code d'étiquette qu'il contient et comparer le code d'étiquette avec les codes d'étiquette associés à l'ordre de préparation de commande numérique ;
i) déposer l'article de pharmacie délivré sur ordonnance et étiqueté dans un bac intermédiaire vide lorsqu'il a été constaté à l'étape de procédé h) que le code d'étiquette correspond à un code d'étiquette qui est enregistré dans l'ordre de préparation de commande numérique ou déposer l'article de pharmacie délivré sur ordonnance et étiqueté dans un bac d'erreur lorsqu'il a été constaté à l'étape de procédé h) que le code d'étiquette ne correspond pas à un code d'étiquette qui est enregistré dans l'ordre de préparation de commande numérique ;
j) répéter les étapes d) à i) pour tous les articles de pharmacie délivrés sur ordonnance qui sont associés à l'ordre de préparation de commande numérique ;
k) distribuer successivement les articles de pharmacie délivrés sur ordonnance à partir des bacs intermédiaires sur un moyen technique de transport, de sorte que chaque article de pharmacie délivré sur ordonnance présente sur le moyen technique de transport au moins une distance prédéterminée par rapport à un autre article de pharmacie délivré sur ordonnance ou à un autre article, et transporter les articles de pharmacie délivrés sur ordonnance au moyen du moyen technique de transport vers un poste d'emballage ;
l) mettre à disposition un carton d'emballage étiqueté, dans lequel l'étiquette du carton d'emballage présente un numéro d'identification de carton, et associer le numéro d'identification de carton à l'ordre de préparation de commande numérique ;
m) scanner le numéro d'identification de carton à partir de l'étiquette du carton d'emballage par un scanner ;
n) distribuer un article de pharmacie délivré sur ordonnance et transporté sur le moyen technique de transport dans une trémie comportant un système de caméras, dans lequel l'article de pharmacie délivré sur ordonnance est scanné sur 6 côtés, dans lequel le code DataMatrix de l'étiquette de l'article de pharmacie délivré sur ordonnance est reconnu et dans lequel le code securPharm de l'article de pharmacie délivré sur ordonnance est reconnu sur l'article de pharmacie délivré sur ordonnance ;
o) comparer, au moyen d'une unité de calcul, le code d'étiquette contenu dans le code DataMatrix scanné et le code securPharm scanné sur l'article de pharmacie délivré sur ordonnance et le numéro d'identification de carton scanné à l'étape de procédé m) avec les données numériques qui sont associées à l'ordre de préparation de commande numérique ;
p) répéter les étapes de procédé n) à o) pour tous les articles de pharmacie délivrés sur ordonnance et distribués sur le moyen technique de transport à l'étape de procédé k), dans lequel tous les articles de pharmacie délivrés sur ordonnance sont distribués dans la même trémie ;
q) distribuer les articles de pharmacie délivrés sur ordonnance et collectés dans la trémie dans le carton d'emballage mis à disposition en ouvrant la trémie ;
r) libérer le carton d'emballage en vue de l'expédition ou d'un traitement ultérieur et de l'annulation des codes securPharm de tous les articles de pharmacie délivrés sur ordonnance et préparés pour une commande auprès du réseau EU-Hub lorsqu'il a été constaté à l'étape de procédé o), pour tous les articles de pharmacie délivrés sur ordonnance à préparer pour une commande, que le code d'étiquette et le code securPharm correspondent à un code d'étiquette et à un code securPharm qui sont enregistrés dans l'ordre de préparation de commande numérique et que le numéro d'identification de carton scanné correspond au numéro d'identification de carton qui est enregistré dans l'ordre de préparation de commande numérique ;
ou transporter le carton d'emballage pour un contrôle d'erreur lorsqu'il a été constaté à l'étape de procédé o), pour au moins un article de pharmacie délivré sur ordonnance à préparer pour une commande, que le code d'étiquette ou le code securPharm ne correspond pas à un code d'étiquette ou à un code securPharm qui sont enregistrés dans l'ordre de préparation de commande numérique ;
ou transporter le carton d'emballage pour un contrôle d'erreur si le numéro d'identification de carton scanné ne correspond pas au numéro d'identification de carton qui est enregistré dans l'ordre de préparation de commande numérique.

2. Procédé selon la revendication 1, **caractérisé en ce que** le procédé comprend en outre la préparation de commandes d'articles de pharmacie et/ou d'articles de droguerie non délivrés sur ordonnance à partir d'un entrepôt, dans lequel un ou plusieurs codes d'identification sont connus pour chaque article de pharmacie et/ou article de droguerie non délivré sur ordonnance, au moyen desquels codes d'identification l'article de pharmacie et/ou l'article de droguerie non délivrés sur ordonnance à préparer pour une commande peuvent être identifiés de manière univoque, et dans lequel au moins un code d'identification est apposé sur chaque article de pharmacie et/ou article de droguerie non délivré sur ordonnance à préparer pour une commande ou sur son emballage extérieur, dans lequel le procédé présente en outre les étapes consistant à
aa) mettre à disposition une ordonnance numérique ou sur papier et créer un ordre de préparation de commande numérique pour l'ordonnance numérique ou sur papier ;
ab) lire l'ordonnance numérique ou sur papier et reconnaître les articles de pharmacie délivrés sur ordonnance commandés par l'ordonnance, les données personnelles figurant sur l'ordonnance électronique, les instructions de dosage, d'autres indications du médecin notées sur l'ordonnance électronique et reconnaître les articles de pharmacie et/ou les articles de droguerie non délivrés sur ordonnance commandés par l'ordonnance ;
ac) associer les données numériques reconnues à l'étape ab) à l'ordre de préparation de commande numérique et, éventuellement, associer d'autres commandes d'articles de pharmacie et/ou d'articles de droguerie non délivrés sur ordonnance ;
ad) éventuellement, mettre à disposition au moins une autre ordonnance numérique ou sur papier et lire l'au moins une autre ordonnance numérique ou sur papier et reconnaître les articles de pharmacie délivrés sur ordonnance commandés par l'au moins une autre ordonnance numérique ou sur papier, les données personnelles figurant sur l'au moins une autre ordonnance numérique ou sur papier, les instructions de dosage, d'autres indications du médecin notées sur l'au moins une autre ordonnance numérique ou sur papier et reconnaître les articles de pharmacie et/ou les articles de droguerie non délivrés sur ordonnance et commandés par l'au moins une autre ordonnance numérique ou sur papier ; et associer les données numériques reconnues à l'ordre de préparation de commande numérique généré à l'étape aa) ;
ae) mettre à disposition un article de pharmacie délivré sur ordonnance, l'ordre de préparation de commande numérique à partir d'un entrepôt au moyen d'un robot et/ou d'un dispositif de transport ;
af) associer le code d'étiquette de l'article de pharmacie délivré sur ordonnance et mis à disposition à l'ordre de préparation de commande numérique ;
ag) imprimer une étiquette présentant des données personnelles du destinataire de l'ordonnance, des indications concernant le dosage de l'article de pharmacie délivré sur ordonnance et mis à disposition, un code DataMatrix présentant le code d'étiquette de l'article de pharmacie délivré sur ordonnance et mis à disposition et, éventuellement, d'autres indications du médecin notées sur une ordonnance électronique et concernant l'article de pharmacie délivré sur ordonnance ;
ah) appliquer l'étiquette sur un côté de l'article de pharmacie délivré sur ordonnance de telle sorte que l'étiquette ne recouvre pas le code securPharm de l'article de pharmacie délivré sur ordonnance ;
ai) scanner le code DataMatrix de l'étiquette et lire le code d'étiquette qu'il contient et comparer le code d'étiquette avec le code d'étiquette associé à l'ordre de préparation de commande numérique ;
aj) déposer l'article de pharmacie délivré sur ordonnance et étiqueté dans un bac intermédiaire vide lorsqu'il a été constaté à l'étape de procédé ai) que le code d'étiquette correspond à un code d'étiquette qui est enregistré dans l'ordre de préparation de commande numérique, ou déposer l'article de pharmacie délivré sur ordonnance et étiqueté dans un bac d'erreur lorsqu'il a été constaté à l'étape de procédé ai) que le code d'étiquette ne correspond pas à un code d'étiquette qui est enregistré dans l'ordre de préparation de commande numérique ;
ak) répéter les étapes ae) à aj) pour tous les articles de pharmacie délivrés sur ordonnance qui sont associés à l'ordre de préparation de commande numérique ;
al) mettre à disposition tous les articles de pharmacie et/ou articles de droguerie non délivrés sur ordonnance contenus dans l'ordre de préparation de commande numérique dans un bac intermédiaire ;
am) distribuer successivement les articles de pharmacie délivrés sur ordonnance à partir des bacs intermédiaires sur un moyen technique de transport, de sorte que chaque article de pharmacie délivré sur ordonnance présente sur le moyen technique de transport au moins une distance prédéterminée par rapport à un autre article de pharmacie délivré sur ordonnance ou à un autre article, et transporter les articles de pharmacie délivrés sur ordonnance au moyen du moyen technique de transport vers un poste d'emballage ;
an) distribuer les articles de pharmacie et/ou articles de droguerie non délivrés sur ordonnance sous forme de tas depuis le bac intermédiaire sur le moyen technique de transport de l'étape de procédé am) ;
ao) mettre à disposition un carton d'emballage étiqueté, dans lequel l'étiquette du carton d'emballage présente un numéro d'identification de carton, et associer le numéro d'identification de carton à l'ordre de préparation de commande numérique ;
ap) scanner le numéro d'identification de carton à partir de l'étiquette du carton d'emballage ;
aq) distribuer un article de pharmacie délivré sur ordonnance et transporté sur le moyen technique de transport dans une trémie, dans lequel l'article de pharmacie délivré sur ordonnance est scanné sur 6 côtés, dans lequel le code DataMatrix de l'étiquette de l'article de pharmacie délivré sur ordonnance est reconnu et dans lequel le code securPharm de l'article de pharmacie délivré sur ordonnance est reconnu sur l'article de pharmacie délivré sur ordonnance ;
ar) comparer le code d'étiquette contenu dans le code DataMatrix scanné et le code securPharm scanné sur l'article de pharmacie délivré sur ordonnance et le numéro d'identification de carton scanné à l'étape de procédé ap) avec les données numériques qui sont associées à l'ordre de préparation de commande numérique ;
as) répéter les étapes de procédé aq) à ar) pour tous les articles de pharmacie délivrés sur ordonnance distribués sur le moyen technique de transport à l'étape de procédé am), dans lequel tous les articles de pharmacie délivrés sur ordonnance sont distribués dans la même trémie ;
at) distribuer le tas d'articles de pharmacie et/ou d'articles de droguerie non délivrés sur ordonnance depuis le moyen technique de transport dans la trémie, dans lequel le tas d'articles de pharmacie et/ou d'articles de droguerie non délivrés sur ordonnance est scanné sur 6 côtés, dans lequel un ou plusieurs codes d'identification de fabricants d'articles de pharmacie et/ou d'articles de droguerie non délivrés sur ordonnance peuvent être reconnus ;
au) comparer chaque code d'identification d'un fabricant d'un article de pharmacie et/ou d'un article de droguerie non délivrés sur ordonnance qui est reconnu à l'étape de procédé at) avec les codes d'identification des fabricants qui sont enregistrés dans l'ordre de préparation de commande numérique ;
av) distribuer les articles de pharmacie délivrés sur ordonnance et les articles de pharmacie et/ou articles de droguerie non délivrés sur ordonnance collectés dans la trémie dans le carton d'emballage mis à disposition ;
aw) fermer et libérer le carton d'emballage pour l'envoi et l'annulation des codes securPharm des articles de pharmacie délivrés sur ordonnance préparés pour une commande auprès du réseau EU-Hub lorsqu'il a été constaté à l'étape de procédé ar), pour tous les articles de pharmacie délivrés sur ordonnance à préparer pour une commande, que le code d'étiquette et le code securPharm correspondent à un code d'étiquette et à un code securPharm enregistrés dans l'ordre de préparation de commande numérique et que le numéro d'identification de carton scanné correspond au numéro d'identification de carton enregistré dans l'ordre de préparation de commande numérique et lorsque tous les codes d'identification de fabricants comparés à l'étape de procédé au) sont enregistrés dans l'ordre de préparation de commande numérique ;
ou transport du carton d'emballage pour un contrôle d'erreur lorsqu'il a été constaté à l'étape de procédé ar), pour au moins un article de pharmacie délivré sur ordonnance à préparer pour une commande, que le code d'étiquette ou le code securPharm ne correspond pas à un code d'étiquette ou à un code securPharm enregistrés dans l'ordre de préparation de commande numérique ;
ou transport du carton d'emballage pour un contrôle d'erreur lorsqu'un code d'identification d'un fabricant comparé à l'étape de procédé au) n'est pas enregistré dans l'ordre de préparation de commande numérique ;
ou transporter le carton d'emballage pour un contrôle d'erreur si le numéro d'identification de carton scanné ne correspond pas au numéro d'identification de carton qui est enregistré dans l'ordre de préparation de commande numérique.

3. Procédé selon l'une des revendications précédentes, **caractérisé en ce que** le poids de chaque article de pharmacie délivré sur ordonnance et de chaque article de pharmacie et article de droguerie non délivré sur ordonnance à préparer pour une commande est en outre connu et **en ce que** les étapes de procédé suivantes sont réalisées pour les cartons d'emballage distribués pour un contrôle à l'étape de procédé r) ou aw)
I. transport du carton d'emballage vers un poste de contrôle ;
II. scannage du numéro d'identification de carton et comparaison de celui-ci avec les données numériques liées à l'ordre de préparation de commande numérique ;
III. déversement du contenu du carton d'emballage dans un récipient ;
IV. réception d'un article de pharmacie délivré sur ordonnance ou d'un article de pharmacie ou d'un article de droguerie non délivré sur ordonnance depuis le récipient par un robot et scannage de l'article de pharmacie sur au moins 6 côtés, dans lequel, dans le cas d'articles de pharmacie délivrés sur ordonnance, le code DataMatrix de l'étiquette et le code securPharm sur l'article de pharmacie délivré sur ordonnance sont scannés et, dans le cas d'articles de pharmacie ou d'articles de droguerie non délivrés sur ordonnance, au moins un code d'identification du fabricant est scanné sur l'article de pharmacie ou l'article de droguerie non délivré sur ordonnance ;
V. comparaison du code d'étiquette et du code securPharm sur l'article de pharmacie délivré sur ordonnance avec les données numériques associées à l'ordre de préparation de commande numérique ; ou comparaison de l'au moins un code d'identification scanné du fabricant avec les données numériques associées à l'ordre de préparation de commande numérique pour un article de pharmacie ou un article de droguerie non délivré sur ordonnance ;
VI. dépôt de l'article de pharmacie délivré sur ordonnance et étiqueté ou de l'article de pharmacie ou de l'article de droguerie délivré sans ordonnance dans le carton d'emballage si, à l'étape V. et à l'étape II., les données scannées correspondent aux données numériques associées à l'ordre de préparation de commande numérique ; ou éjection dans un bac d'erreur si, à l'étape V. ou à l'étape II., les données scannées ne correspondent pas aux données numériques de l'ordre de préparation de commande numérique ;
VII. répétition des étapes IV. à VI. pour tous les articles de pharmacie délivrés sur ordonnance, les articles de pharmacie et articles de droguerie non délivrés sur ordonnance déversés à l'étape de procédé III. ;
VIII. transport du carton d'emballage sur un moyen technique de transport vers une balance et pesée du carton d'emballage si, à l'étape de procédé VI., aucun article de pharmacie et/ou article de droguerie n'a été éjecté dans un bac d'erreur, dans lequel le poids déterminé est le poids réel du carton d'emballage ;
IX. calcul du poids de consigne du carton d'emballage et comparaison du poids réel du carton d'emballage avec le poids de consigne du carton d'emballage ;
X. fermeture et libération du carton d'emballage en vue de l'expédition si, à l'étape IX., un écart entre le poids de consigne et le poids réel est constaté à l'intérieur d'une plage de tolérance prédéfinie ou distribution du carton d'emballage sur un moyen technique de transport pour un contrôle supplémentaire si, à l'étape IX., un écart entre le poids de consigne et le poids réel est constaté en dehors d'une plage de tolérance prédéfinie.

4. Procédé selon l'une des revendications précédentes, **caractérisé en ce que** les articles de pharmacie délivrés sur ordonnance sont de préférence de forme parallélépipédique.

5. Procédé selon la revendication 3, **caractérisé en ce qu'**entre 0 % et 100 % des cartons d'emballage préparés pour une commande, de préférence entre 0 % et 25 % des cartons d'emballage préparés pour une commande, de manière particulièrement préférée entre 0 % et 5 % des cartons d'emballage préparés pour une commande sont distribués sur le moyen technique de transport pour un contrôle supplémentaire après l'étape de procédé r) ou aw), dans lequel les cartons d'emballage distribués sont prévus pour l'expédition ou le traitement ultérieur.

6. Procédé selon l'une des revendications précédentes, **caractérisé en ce que,** à l'étape de procédé n), at) ou aq), une photo ou plusieurs photos des articles de pharmacie à préparer pour une commande et/ou du contenu du carton d'emballage sont en outre prises.

7. Procédé selon l'une des revendications précédentes,
**caractérisé en ce qu'**une photo ou plusieurs photos du contenu d'un carton d'emballage complet préparé pour une commande sont prises avant que celui-ci ne soit fermé pour l'expédition.

8. Procédé selon l'une des revendications précédentes, **caractérisé en ce que** le code securPharm de chacun des articles de pharmacie délivrés en pharmacie et préparés pour une commande dans un carton d'emballage est automatiquement annulé dans la base de données securPharm lors de l'expédition du carton, dans lequel un horodatage est généré pour l'annulation dans la base de données securPharm.

9. Procédé selon la revendication 8, **caractérisé en ce que** les données de l'étiquette et l'horodatage pour l'annulation sont liés et mémorisés entre eux et avec l'ordre de préparation de commande numérique dans la base de données securPharm.

10. Procédé selon l'une des revendications précédentes, **caractérisé en ce que** le procédé comprend en outre le tri d'articles de pharmacie délivrés sur ordonnance.

11. Procédé selon la revendication 10, **caractérisé en ce que** le procédé présente en outre les étapes consistant à
• mettre à disposition une pluralité d'articles de pharmacie délivrés sur ordonnance ;
• séparer la pluralité d'articles de pharmacie délivrés sur ordonnance sur un moyen technique de transport, de sorte que chacun des articles de pharmacie délivrés sur ordonnance présente au moins une distance prédéterminée par rapport à un autre article de pharmacie délivré sur ordonnance ;
• scanner chacun des articles de pharmacie délivrés sur ordonnance sur 6 côtés ;
• reconnaître le code securPharm sur chacun des articles de pharmacie délivrés sur ordonnance scannés et sa position sur chacun des articles de pharmacie délivrés sur ordonnance scannés ;
• mémoriser le ou les codes d'identification d'un fabricant ainsi que la position du code securPharm sur un article de pharmacie délivré sur ordonnance pour l'article de pharmacie délivré sur ordonnance respectivement scanné et, éventuellement, créer un code d'étiquette pour chaque article de pharmacie délivré sur ordonnance scanné ;
• stocker dans un entrepôt les articles de pharmacie délivrés sur ordonnance pour lesquels le code securPharm ne se trouve pas sur le côté supérieur de l'article de pharmacie délivré sur ordonnance ;
• distribuer dans un bac d'erreur l'article de pharmacie délivré sur ordonnance pour lequel le code securPharm se trouve sur le côté supérieur de l'article de pharmacie délivré sur ordonnance.

12. Dispositif pour la mise en œuvre du procédé selon l'une des revendications 1 à 11, présentant
• au moins une unité de calcul ;
• au moins une machine à étiqueter ;
• au moins un moyen technique de transport relié à un entrepôt ;
• une trémie comportant un système de caméras, dans lequel le système de caméras présente au moins 6 caméras ;
• au moins un second moyen technique de transport ;
• au moins un scanner ;
• éventuellement, au moins une balance ;
• éventuellement, au moins un robot comportant une unité de préhension.

13. Dispositif selon la revendication 12, **caractérisé en ce que** le dispositif comprend en outre
• au moins un séparateur ;
• au moins un autre moyen technique de transport ;
• au moins un poste où un article de pharmacie délivré sur ordonnance peut être scanné sur 6 côtés ;
• un entrepôt.
